# EUROPEAN PATENT SPECIFICATION

(11) **EP 4 452 116 B1**
(45) Date of publication and mention of the grant of the patent: **08.04.2026**
(21) Application number: 22851192.9
(22) Date of filing: 16.12.2022
(51) Int. Cl.: A61B 34/10

(54) **PLANNING METHODS FOR MODIFYING TOOL OPERATION BASED ON TISSUE PARAMETERS**
PLANUNGSVERFAHREN ZUR MODIFIZIERUNG DES WERKZEUGBETRIEBS AUF BASIS VON GEWEBEPARAMETERN
PROCÉDÉS DE PLANIFICATION OUR MODIFIER UNE OPÉRATION D'OUTIL SUR LA BASE DE PARAMÈTRES DE TISSU

(30) Priority: 20.12.2021 US 202163291566 P
(43) Date of publication of application: 30.10.2024
(60) Divisional application: 26155322.6
(73) Proprietor: MAKO Surgical Corp., Weston, FL 33331 (US)
(72) Inventor: KHURANA, Rishabh, Fort Lauderdale, FL 33301 (US); WILLIAMS, Brandt, Plantation, FL 33325 (US); MUNIZ, Erick, Saint Joseph, MI 49085 (US); SHEN, Delin, Wayland, MA 01778 (US)
(74) Representative: Schott, Jakob Valentin
(86) International application number: PCT/US2022/053172
(87) International publication number: WO 2023/121966

(56) References cited:
- US-A1- 2019 090 966
- US-A1- 2019 380 788

## Description

### CROSS-REFERENCE TO RELATED APPLICATIONS

The subject application claims priority to and all the benefits of United States Provisional Patent Application No. 63/291,566, filed December 20, 2021.

### TECHNICAL FIELD

The present disclosure relates generally to robotic systems, methods, and software programs for modifying tool operation based on tissue parameters, such as bone density.

### BACKGROUND

Robotic systems that perform surgical procedures at surgical sites often include a manipulator with a base, a plurality of links and joints, and an end effector coupled to the manipulator. Many times, the end effector comprises or supports a surgical tool with an energy applicator designed to remove tissue at the surgical site.

The energy applicator is typically moved along a tool path to remove tissue at the surgical site. The velocity or speed at which the energy applicator moves along the tool path is commonly known to those skilled in the art as a "feed rate" of the tool or energy applicator.

Prior systems have contemplated to adjust or determine the feed rate to account for conditions or inputs, such as manual user selection of the feed rate, characteristics of the tissue such as bone mineral density, sensed forces applied to the energy applicator, curvature of the path, and the like.

However, prior feed rate techniques fall short in providing in-depth pre-planning of the tool path feed rates based on, at least, detailed analysis of the patient imaging data and density of the anatomy relative to the planned tool path. For example, in determining the feed rate, the prior techniques fail to account for, among other things, a ratio of intersection of the tool geometry relative to the anatomy as evaluated at point(s) along the tool path or whether certain portions of the anatomy would have been already removed by the tool at another point along the tool path. In turn, the prior feed rate techniques may lead to outcomes that are less than optimal, such as implant fit issues due to resection under-sizing or tool skiving, formation of pits in the resection surface, sub-optimal cutting forces applied by the tool, and the like. There remains a need to address at least some of these deficiencies. US 2019/090966 A1 discloses a robotic system and methods for performing spine surgery. US 2019/0380788 discloses systems, methods, software and techniques for generating a milling path for a tool of a surgical system.

### SUMMARY

This Summary introduces a selection of concepts in a simplified form that are further described below in the Detailed Description below. The invention is defined by the feature combination of claim 1. Preferable embodiments are defined in the dependent claims.

In a first aspect according to the presently claimed invention, a computer-implemented surgical planning method is provided. The method comprises: obtaining anatomical data comprising a geometry and density values of an anatomical volume; obtaining path data comprising a tool path along which a robotic manipulator will move a tool to interact with the anatomical volume, the tool path defined by points between which the tool will successively pass; obtaining tool data including a geometry of the tool that will interact with the tool path; merging the path data and the anatomical data; and for at least one point of the tool path: identifying a location of the point relative to the anatomical data, loading, from the tool data, the geometry of the tool at the identified location, at the identified location, identifying an intersection between the geometry of the tool and the anatomical volume, determining density values of the anatomical data within the intersection, computing a tool contact factor related to an interaction between the geometry of the tool and the anatomical volume, setting a planned feed rate factor for the tool based on the determined density values and the computed tool contact factor, and associating the planned feed rate factor with the at least one point; and outputting cut plan data comprising the tool path including the planned feed rate factor associated with the at least one point of the tool path.

In a second aspect not according to the presently claimed invention, a non-transitory computer readable medium is provided. The non-transitory computer readable medium has stored thereon instructions which, when executed by one or more processors, implements a computer-implemented surgical planning software configured to: obtain anatomical data comprising a geometry and density values of an anatomical volume; obtain path data comprising a tool path along which a robotic manipulator will move a tool to interact with the anatomical volume, the tool path defined by points between which the tool will successively pass; obtain tool data including a geometry of the tool that will interact with the tool path; merge the path data and the anatomical data; and for at least one point of the tool path: identify a location of the point relative to the anatomical data, load, from the tool data, the geometry of the tool at the identified location, at the identified location, identify an intersection between the geometry of the tool and the anatomical volume, determine density values of the anatomical data within the intersection, compute a tool contact factor related to an interaction between the geometry of the tool and the anatomical volume, set a planned feed rate factor for the tool based on the determined density values and the computed tool contact factor, and associate the planned feed rate factor with the at least one point; and output cut plan data comprising the tool path including the planned feed rate factor associated with the at least one point of the tool path.

In a third aspect not according to the presently claimed invention, a surgical system is provided. The surgical system comprising: a robotic manipulator configured to support and move a tool; and a control system coupled to the robotic manipulator, and comprising: one or more processors; and a non-transitory computer readable medium, having stored thereon instructions which, when executed by the one or more processors, implement a computer-implemented surgical planning software configured to: obtain anatomical data comprising a geometry and density values of the anatomical volume; obtain path data comprising a tool path along which a robotic manipulator will move a tool to interact with an anatomical volume, the tool path defined by points between which the tool will successively pass; obtain tool data including a geometry of the tool that will interact with the tool path; merge the path data and the anatomical data; and for at least one point of the tool path: identify a location of the point relative to the anatomical data, load, from the tool data, the geometry of the tool at the identified location, at the identified location, identify an intersection between the geometry of the tool and the anatomical volume, determine density values of the anatomical data within the intersection, compute a tool contact factor related to an interaction between the geometry of the tool and the anatomical volume, set a planned feed rate factor for the tool based on the determined density values and the computed tool contact factor, and associate the planned feed rate factor with the at least one point; and output cut plan data comprising the tool path including the planned feed rate factor associated with the at least one point of the tool path; and wherein the control system utilizes the outputted cut plan data to control the robotic manipulator to move the tool along the tool path according to the planned feed rate factor associated with the at least one point of the tool path for enabling the tool to interact with the anatomical volume. A method of operating the surgical system of the third aspect is also provided.

In a fourth aspect not according to the presently claimed invention, a computer-implemented surgical planning method is provided. The computer-implemented surgical planning method comprises: obtaining anatomical data comprising a geometry of an anatomy; obtaining path data comprising a tool path along which a robotic manipulator will move a tool to interact with the anatomy, the tool path defined by points between which the tool will pass; obtaining tool data including a geometry of the tool; merging the path data and the anatomical data; for at least one point of the tool path: identifying a location of the point relative to the anatomical data, loading, from the tool data, the geometry of the tool at the identified location, computing a tool contact factor related to an interaction between the geometry of the tool and the anatomical volume, setting a planned feed rate factor for the tool based on the computed tool contact factor, and associating the planned feed rate factor with the at least one point; and outputting cut plan data comprising the tool path including the planned feed rate factor associated with the at least one point of the tool path. A non-transitory computer readable medium, or computer program product, comprising instructions to execute the computer-implemented method of the fourth aspect is also provided.

In a fifth aspect not according to the presently claimed invention, a computer-implemented method is provided. The computer-implemented method comprises: obtaining anatomical data comprising a geometry and density values of an anatomical volume; obtaining virtual object data comprising geometry of a virtual object having a mesh of polygonal elements; merging the anatomical data and the virtual object data; determining a parameter for one or more polygonal elements of the mesh based on the density values; and modifying operation of a tool according to the parameter in response to the tool interacting with the one or more polygonal elements. A non-transitory computer readable medium, or computer program product, comprising instructions to execute the computer-implemented method of the fifth aspect is also provided.

In a sixth aspect not according to the presently claimed invention, a computer-implemented method is provided. The computer-implemented method comprises: obtaining anatomical data comprising a geometry and density values of an anatomical volume; obtaining virtual object data comprising geometry of a virtual object having a mesh of polygonal elements; and merging the anatomical data and the virtual object data to determine a parameter for one or more polygonal elements of the mesh based on the density values. A non-transitory computer readable medium, or computer program product, comprising instructions to execute the computer-implemented method of the sixth aspect is also provided.

In a seventh aspect not according to the presently claimed invention, a non-transitory computer readable medium is provided. The non-transitory computer readable medium has stored thereon instructions which, when executed by one or more processors, implements a computer-implemented surgical planning software configured to: obtain anatomical data comprising a geometry and a tissue parameter of an anatomical volume; obtain path data comprising a tool path along which a robotic manipulator will move a tool to interact with the anatomical volume, the tool path defined by points between which the tool will successively pass; obtain tool data including a geometry of the tool that will interact with the tool path; merge the path data and the anatomical data; and for at least one point of the tool path: identify a location of the point relative to the anatomical data, load, from the tool data, the geometry of the tool at the identified location, at the identified location, identify an intersection between the geometry of the tool and the anatomical volume, determine the tissue parameter of the anatomical data within the intersection, compute a tool contact factor related to an interaction between the geometry of the tool and the anatomical volume, set a planned feed rate factor for the tool based on the determined tissue parameter and the computed tool contact factor, and associate the planned feed rate factor with the at least one point; and output cut plan data comprising the tool path including the planned feed rate factor associated with the at least one point of the tool path. A computer-implemented method of executing the steps of the seventh aspect is also provided.

In an eighth aspect not according to the presently claimed invention, a non-transitory computer readable medium is provided. The non-transitory computer readable medium has stored thereon instructions which, when executed by one or more processors, implements a computer-implemented surgical planning software configured to: obtain anatomical data comprising a geometry and a tissue parameter of an anatomical volume; obtain path data comprising a tool path along which a robotic manipulator will move a tool to interact with the anatomical volume, the tool path defined by points between which the tool will successively pass; obtain tool data including a geometry of the tool that will interact with the tool path; merge the path data and the anatomical data; and for at least one point of the tool path: identify a location of the point relative to the anatomical data, load, from the tool data, the geometry of the tool at the identified location, at the identified location, identify an intersection between the geometry of the tool and the anatomical volume, determine the tissue parameter of the anatomical data within the intersection, compute a tool contact factor related to an interaction between the geometry of the tool and the anatomical volume, set a output pose for the tool based on the determined tissue parameter and the computed tool contact factor, and associate the output pose with the at least one point; and output cut plan data comprising the tool path including the output pose associated with the at least one point of the tool path. A computer-implemented method of executing the steps of the eighth aspect is also provided.

In a ninth aspect not according to the presently claimed invention, a non-transitory computer readable medium is provided. The non-transitory computer readable medium has stored thereon instructions which, when executed by one or more processors, implements a computer-implemented surgical planning software configured to: obtain anatomical data comprising a geometry and a tissue parameter of an anatomical volume; obtain path data comprising a tool path along which a robotic manipulator will move a tool to interact with the anatomical volume, the tool path defined by points between which the tool will successively pass; obtain tool data including a geometry of the tool that will interact with the tool path; merge the path data and the anatomical data; and for at least one point of the tool path: identify a location of the point relative to the anatomical data, load, from the tool data, the geometry of the tool at the identified location, at the identified location, identify an intersection between the geometry of the tool and the anatomical volume, determine the tissue parameter of the anatomical data within the intersection, compute a tool contact factor related to an interaction between the geometry of the tool and the anatomical volume, set a planned feed rate factor for the tool based on the determined tissue parameter, the computed tool contact factor, and achieving a predetermined motor current on a motor of the tool, and associate the planned feed rate factor with the at least one point; and output cut plan data comprising the tool path including the planned feed rate factor associated with the at least one point of the tool path. A computer-implemented method of executing the steps of the ninth aspect is also provided.

In a tenth aspect not according to the presently claimed invention,
a non-transitory computer readable medium, having stored thereon instructions which, when executed by one or more processors, implement a computer-implemented surgical planning software configured to: obtain anatomical data comprising a geometry and a tissue parameter of an anatomical volume; obtain path data comprising a tool path along which a robotic manipulator will move a tool to interact with the anatomical volume, the tool path defined by points between which the tool will successively pass; obtain tool data including a geometry of the tool that will interact with the tool path; merge the path data and the anatomical data; and for at least one point of the tool path: identify a location of the point relative to the anatomical data, load, from the tool data, the geometry of the tool at the identified location, at the identified location, identify an intersection between the geometry of the tool and the anatomical volume, determine the tissue parameter of the anatomical data within the intersection, compute a tool contact factor related to an interaction between the geometry of the tool and the anatomical volume, set a tool operation parameter based on the determined tissue parameter and the computed tool contact factor, and associate the tool operation parameter with the at least one point; and output plan data comprising the tool path including the tool operation parameter associated with the at least one point of the tool path. A computer-implemented method of executing the steps of the tenth aspect is also provided.

In an eleventh aspect not according to the presently claimed invention, a computer-implemented method is provided comprising: identifying an intersection between a geometry of a tool and an anatomical volume, determining a tissue parameter of the anatomical data within the intersection, computing a tool contact factor related to an interaction between the geometry of the tool and the anatomical volume, and setting a tool operation parameter based on the determined tissue parameter and the computed tool contact factor. A computer program product or robotic system implementing the steps of the eleventh aspect is also provided.

Any of the above aspects can be combined in part or in whole.

Any of the above aspects can be implemented as a computer-implemented method, a robotic surgical system, and/or a non-transitory computer readable medium having stored thereon instructions which, when executed by one or more processors, implement a computer-implemented surgical planning software or computer program product.

Any of the above aspects can be utilized with any of the following implementations, whether used in part or in whole.

In one implementation, obtaining anatomical data includes obtaining a bone model associated with the anatomical volume. In one implementation, obtaining path data further includes obtaining the tool path being predetermined based on a planned resection volume of the bone model of the anatomical volume. In one implementation, obtaining path data further includes obtaining the tool path being predetermined based on a geometry of an implant model selected for the bone model. In some implementations, the bone model may be patient-specific or a statistical bone model. In some implementations, obtaining path data includes obtaining a predefined pose of the tool with respect to at least one point of the tool path. The bone model can be of an acetabulum, a glenoid, or any type of joint socket. The bone model can be of a femur, tibia, humerus, or portion of the spine (vertebra). In one implementation, the anatomical data is obtained without preoperative images (e.g., imageless) or without forming a bone model. The bone model may be patient-specific or may be based on a statistical dataset from a similar population.

In one implementation, obtaining anatomical data includes obtaining imaging data including slices of the anatomical volume. In one implementation, obtaining imaging data includes obtaining DICOM data including intercept and slope values, slice thickness, and patient position at a time of imaging. In one implementation, obtaining anatomical data includes obtaining CT slices of the anatomical volume. In such an implementation, the geometry of the tool comprises a 3-D geometry of the tool and, for the at least one point of the tool path, loading the 3-D geometry of the tool at the identified location, and at the identified location, identifying CT slices for which there is a cross-sectional intersection between the 3-D geometry of the tool and the anatomical volume; for each identified CT slice, determining a Hounsfield unit for each pixel within the cross-sectional intersection; and collecting the Hounsfield units from the pixels within the cross-sectional intersections of each of the identified CT slices.

In one implementation, identifying the intersection, at the identified location, between the geometry of the tool and the anatomical volume includes identifying one or more slices of the imaging data exhibiting the intersection between the geometry of the tool and the anatomical volume. One implementation includes, for the at least one point of the tool path, and after identifying slices of the imaging data exhibiting the intersection between the geometry of the tool and the anatomical volume, for each identified slice, computing the intersection ratio related to the geometry of the tool within the intersection relative to the geometry of the tool outside of the intersection. One implementation includes, for each identified slice, determining a quantity of pixels within the geometry of the tool having Hounsfield units exceeding a predetermined threshold.

In one implementation, determining density values of the anatomical data within the intersection includes, for each identified slice, determining radiodensity values of the imaging data located within the intersection. In one implementation, determining density values of the anatomical data within the intersection includes collecting radiodensity values located within the intersections of each of the one or more identified slices. In one implementation collecting the radiodensity values located within the intersection of each of the one or more identified slices includes identifying one or more of: an average, a median, and a maximum radiodensity value from the collected radiodensity values.

In one implementation, setting the planned feed rate factor for the tool based on the determined density values includes calculating a bone mineral density (BMD) factor of the anatomical volume relative to the identified location based on the collected radiodensity values. In one implementation, setting the planned feed rate factor for the tool based on the determined density values includes setting the planned feed rate factor for the tool based on the calculated BMD factor. In one implementation, calculating the BMD factor includes converting an average, a median, and/or a maximum radiodensity value into the BMD factor. In one implementation, converting the one or more of: the average, the median, and the maximum radiodensity value into the BMD factor includes multiplying the one or more of: the average, the median, and the maximum radiodensity value with the intersection ratio. In one implementation, setting the planned feed rate factor for the tool based on the determined density values includes setting the planned feed rate factor based on the calculated BMD factor. One implementation includes accessing a look-up table defining associations between predefined bone mineral density (BMD) factors and predefined feed rate factors. In such an implementation, for at least one point of the tool path, setting the planned feed rate factor based on the calculated BMD factor includes identifying, in the look-up table, the predefined BMD factor that is closest to the calculated BMD factor; and setting the planned feed rate factor based on the predefined feed rate factor associated with the closest identified predefined BMD factor in the look-up table. In one implementation, for the at least one point of the tool path, setting the planned feed rate factor based on the calculated BMD factor includes setting the planned feed rate factor to be a maximum feed rate factor in response to determining that the calculated BMD factor is below a minimum threshold; and/or setting the planned feed rate factor to be a minimum feed rate factor in response to determining that the calculated BMD factor is above a maximum threshold. In one implementation, setting the planned feed rate factor includes setting the planned feed rate factor based on achieving a predetermined motor current on a motor of the tool.

One implementation includes, for one point of the tool path, storing interaction coordinates obtained from the intersection between the geometry of the tool and the anatomical volume at the location of the one point, the interaction coordinates indicative of locations of simulated interaction between the geometry of the tool and the anatomical volume at the location of the one point; and for a second point of the tool path successive to the one point; identifying a location of the second point relative to the anatomical data, loading, from the tool data, the geometry of the tool at the identified location of the second point, at the identified location of the second point, identifying an intersection between the geometry of the tool and the anatomical volume, determining density values of the anatomical data within the intersection at the location of the second point, comparing coordinates of the determined density values to the interaction coordinates; and disregarding any density values having coordinates that are identical to the interaction coordinates.

One implementation includes, for the at least one point of the tool path, disregarding any density values located beyond the intersection between the geometry of the tool and the anatomical volume.

In one implementation, merging the path data and the anatomical data includes merging the path data and anatomical data into a common coordinate system.

In one implementation, the cut plan data comprises the tool path along which a robotic manipulator will move a tool in an autonomous mode to interact with the anatomical volume.

One implementation includes, for each point of the tool path: identifying the location of the point relative to the anatomical data, loading, from the tool data, the geometry of the tool at the identified location, at the identified location, identifying the intersection between the geometry of the tool and the anatomical volume, determining density values of the anatomical data within the intersection, setting the planned feed rate factor for the tool based on the determined density values, and associating the planned feed rate factor with the point; and wherein outputting the cut plan data further comprises the tool path including the planned feed rate factor associated with each point of the tool path.

In one implementation, the robotic manipulator is commanded to move the tool according to a first feed rate; while the tool moves according to the first feed rate, an interaction between the virtual model and the one or more polygonal elements of the virtual object is detected. A second feed rate is computed based on the parameter associated with the one or more polygonal elements. Operation of the tool is modified such that the robotic manipulator is commanded to move the tool at the second feed rate.

In one implementation, the robotic manipulator is commanded to move the tool. The virtual model of the tool penetrates the one or more polygonal elements of the virtual object; A reactive force is computed based on the parameter associated with the one or more polygonal elements; a reactive force is applied to the virtual model in the virtual simulation to reduce penetration of the one or more polygonal elements by the virtual model; and operation of the tool is modified such that the robotic manipulator is commanded to move the tool in accordance with application of the reactive force to the virtual model in the virtual simulation to constrain movement of the tool relative to the virtual object.

### DESCRIPTION OF THE DRAWINGS

Advantages of the present disclosure will be readily appreciated as the same becomes better understood by reference to the following detailed description when considered in connection with the accompanying drawings.
Figure 1 is a perspective view of one implementation of a robotic surgical system.
Figure 2 is a block diagram of a control system, including components and controllers, for controlling the robotic surgical system, according to one implementation.
Figure 3 is a functional block diagram of a software program utilized by the robotic surgical system, according to one implementation.
Figure 4 is a flow chart of a method determining cut plan data.
Figure 5 is a diagram illustrating a step of the method of Figure 4 of merging path data and anatomical data.
Figure 6 is a diagrammatic view of cut plan data.
Figure 7 includes multiple cross-sectional views of an intersection of a tool of the robotic surgical system of Figure 1 and an anatomical volume.
Figure 8 is a cross-sectional view of an intersection of the tool of the robotic surgical system of Figure 1 and the anatomical volume.
Figure 9 is a table illustrating stored quantities used for determining the cut plan data.
Figure 10 is a look-up table defining associations between predefined bone mineral density factors and predefined feed rate factors.
Figure 11 is a cross-sectional view of an intersection of the tool of the robotic surgical system of Figure 1 and the anatomical volume when the tool is located at a first point and an intersection of the tool of the robotic surgical system of Figure 1 and the anatomical volume when the tool is located at a second point.
Figure 12 is a diagram illustrating a step of a virtual object and anatomical data.
Figures 13A and 13B are various view of the tool of the robotic surgical system of Figure 1 interacting with the virtual object of Figure 12.

### DETAILED DESCRIPTION

### I. System Overview

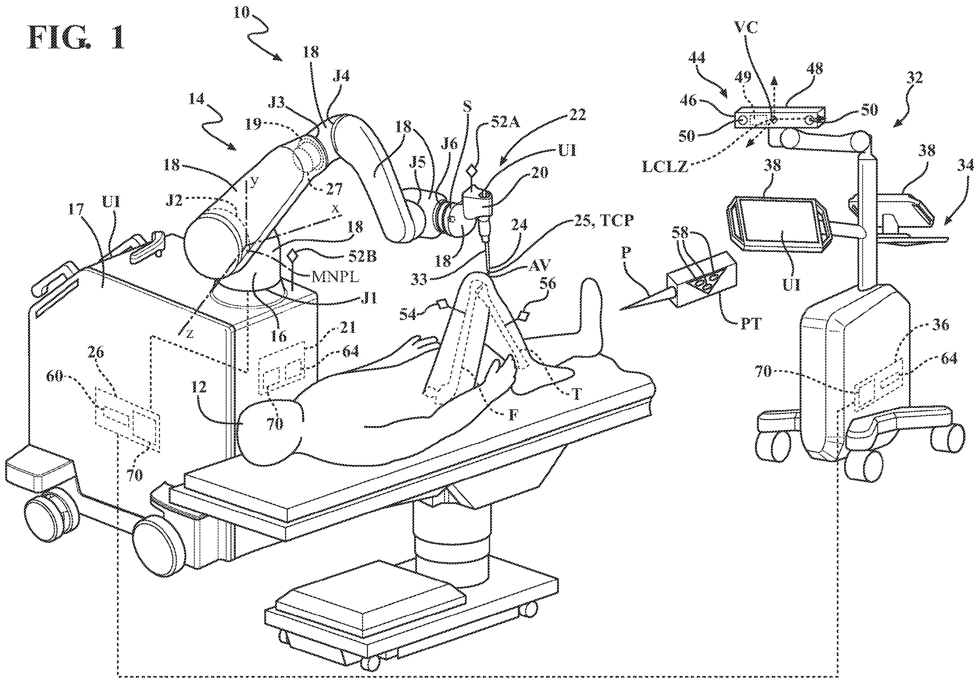

Referring to FIG. 1, a robotic surgical system 10 is illustrated. The system 10 is useful for treating a surgical site or anatomical volume (AV) of a patient 12, such as treating bone or soft tissue. In FIG. 1, the patient 12 is undergoing a surgical procedure. The anatomy in FIG. 1 includes a femur F and a tibia T of the patient 12. The surgical procedure may involve tissue removal or other forms of treatment. Treatment may include cutting, coagulating, lesioning the tissue, other *in-situ* tissue treatments, or the like. In some examples, the surgical procedure involves partial or total knee or hip joint replacement surgery, shoulder joint replacement surgery, spine surgery, or ankle surgery. In some examples, the system 10 is designed to cut away material to be replaced by surgical implants, such as hip and knee implants, including unicompartmental, bicompartmental, multicompartmental, or total knee implants. Some of these types of implants are shown in U.S. Patent Application Publication No. 2012/0330429, entitled "Prosthetic Implant and Method of Implantation". The system 10 and techniques disclosed herein may be used to perform other procedures, surgical or non-surgical, or may be used in industrial applications or other applications where robotic systems are utilized.

As shown in FIG. 1, the system 10 includes a manipulator 14. A manipulator cart 17 (as shown in FIG. 1) can support the manipulator 14 such that the manipulator 14 is fixed to the manipulator cart 17. The manipulator 14 has a base 16 and plurality of links 18. In one example, pairs of adjacent links 18 are connected by one of the joints J. The links 18 collectively form one or more arm(s) 23 of the manipulator 14. The manipulator 14 may have a serial arm configuration (as shown in FIG. 1), a parallel arm configuration, or any other suitable manipulator configuration. In other examples, more than one manipulator 14 may be utilized in a multiple arm configuration. The manipulator 14 according to one example has six joints J1-J6 implementing at least six-degrees of freedom (DOF) for the manipulator 14. The manipulator 14 may have any number of degrees of freedom and may have any suitable number of joints J and may have redundant joints. The manipulator 14 can also be mounted to a surgical table or partially supported by the patient.

At each joint J, there may be an actuator, such as a joint motor 27 disposed between adjacent links 18. The joint motors 27 are configured to rotate the links 18. As such, positions of the links 18 are set by joint motors 27. Each joint motor 27 may be attached to a structural frame internal to the manipulator 14. In one example, the joint motor 27 is a servo motor, such as a permanent magnet brushless motor. The joint motor 27 may have other configurations, such as synchronous motors, brush-type DC motors, stepper motors, induction motors, and the like.

The joint motors 27 are positioned at one of a plurality of angular positions, hereinafter referred to as joint angles. The joint angle is the angle of the joint J between adjacent links 18. Each joint J may be configured to undergo a joint torque. The joint torque is a turning or twisting "force" of the joint J and is a function of the force applied at a length from a pivot point of the joint J. A torque sensor may be connected to one or more joint motors 27 for measuring the joint torque of the joint J. Alternatively, signals representative of currents applied to the joint motors 27 may be used to measure the joint torques.

One or more joint motors 27 may be equipped with a position sensor or encoder 19. For simplicity, one joint encoder 19 is illustrated in FIG. 1, although other joint encoders 19 may be similarly illustrated. Alternatively, one or more links 18 being driven by that joint motor 27 may be equipped with the position sensor or encoder 19. The encoder 19 may measure the joint angle of the respective joint J. In some embodiments, two encoders, one for the joint motor 27 and one for the link 18 being moved can be used to determine the joint angle, such as by averaging the joint angle, and the displacement between joint motor 27 and joint through the compliant transmission. The manipulator 14 need not require joint encoders 19 but may alternatively, or additionally, utilize motor encoders present on joint motors 27 at one or more joints J. Also, the manipulator 14 need not require rotary joints, but may alternatively, or additionally, utilize one or more prismatic joints. Certain joints J may be passively moveable and lockable while other joints J may be actively driven. Any suitable combination of joint types is contemplated.

Referring to FIG. 1, the base 16 of the manipulator 14 is generally a portion of the manipulator 14 that provides a fixed reference coordinate system for other components of the manipulator 14 or the system 10 in general. Generally, the origin of a manipulator coordinate system MNPL is defined at the fixed reference of the base 16. The base 16 may be defined with respect to any suitable portion of the manipulator 14, such as one or more of the links 18. Alternatively, or additionally, the base 16 may be defined with respect to the manipulator cart 17, such as where the manipulator 14 is physically attached to the manipulator cart 17. In one example, the base 16 is defined at an intersection of the axes of joints J1 and J2. Thus, although joints J1 and J2 are moving components in reality, the intersection of the axes of joints J1 and J2 is nevertheless a virtual fixed reference pose, which provides both a fixed position and orientation reference and which does not move relative to the manipulator 14 and/or manipulator cart 17. In other examples, the manipulator 14 can be a hand-held manipulator where the base 16 is a base portion of a tool (e.g., a portion held free hand by the user or coupled to a set-up linkage) and the tool tip is movable (e.g., semi-autonomously) relative to the base portion. The base portion has a reference coordinate system that is tracked, and the tool tip has a tool tip coordinate system that is computed relative to the reference coordinate system (e.g., via motor and/or joint encoders and forward kinematic calculations). Movement of the tool tip can be controlled to follow the path since its pose relative to the path can be determined. Such a hand-held configuration can be like that described in U.S. Patent No. 9,707,043, entitled "Surgical Instrument Including Housing, a Cutting Accessory that Extends from the Housing and Actuators that Establish the Position of the Cutting Accessory Relative to the Housing".

A tool 20 couples to the manipulator 14 and is movable relative to the base 16 to interact with the anatomy in certain modes. The tool 20 is a physical and surgical tool and is, or forms part of, an end effector 22 supported by the manipulator 14 in certain embodiments. The tool 20 may be grasped by the user. One possible arrangement of the manipulator 14 and the tool 20 is described in U.S. Patent No. 9,119,655, entitled "Surgical Manipulator Capable of Controlling a Surgical Instrument in Multiple Modes". The manipulator 14 and the tool 20 may be arranged in alternative configurations. The tool 20 can be like that shown in U.S. Patent Application Publication No. 2014/0276949, filed on March 15, 2014, entitled "End Effector of a Surgical Robotic Manipulator".

The tool 20 includes an energy applicator 24 designed to contact and remove the tissue of the patient 12 at the surgical site. In one example, the energy applicator 24 is a bur 25 or surgical cutter. The tool 20 may comprise a tool shaft 33 having a proximal end coupled to the manipulator 14 and a distal end where the energy applicator 24 is located. The tool shaft 33 rotates about a cutting axis such that the energy applicator 24 can manipulate the tissue. The bur 25 may be substantially spherical and comprise a spherical center, radius (r) and diameter. Alternatively, the energy applicator 24 may be a drill bit, a saw blade, an ultrasonic vibrating tip, or the like. The tool 20 and/or energy applicator 24 may comprise any geometric feature, e.g., perimeter, circumference, radius, diameter, width, length, volume, area, surface/plane, range of motion envelope (along any one or more axes), etc. The geometric feature may be considered to determine how to locate the tool 20 relative to the tissue at the surgical site to perform the desired treatment. In some of the embodiments described herein, a spherical bur having a tool center point (TCP) will be described for convenience and ease of illustration but is not intended to limit the tool 20 to any particular form.

A sensor S, such as a force-torque sensor, may be mounted proximal the end effector 22. The force-torque sensor S is configured to output variable signals as a function of a force and/or a torque to which the end effector 22 and/or tool 20 are exposed. By doing so, the force-torque sensor S allows sensing of an input force applied to the end effector 22 and/or tool 20 by the user. The input force can be utilized to control movement of the manipulator 14 to emulate the user's applied force/torque. The force-torque sensor S may also sense external forces applied to the energy applicator 24. In one implementation, the force-torque sensor S is a 6DOF sensor such that the force-torque sensor S is configured to output signals representative of three mutually orthogonal forces and three torques about the axes of the orthogonal forces that are applied to the tool 20. Additionally, or alternatively, the input force applied to the end effector 22 and/or tool 20 may be determined using joint torques or electrical current sensors on the joint motors 27.

Referring to FIG. 1, the manipulator 14 and/or manipulator cart 17 may house a manipulator controller 26, or other type of control unit. The manipulator controller 26 may comprise one or more computers, or any other suitable form of controller that directs the motion of the manipulator 14. The manipulator controller 26 may have a central processing unit (CPU) and/or other processors, memory (not shown), and storage (not shown). The manipulator controller 26 is loaded with software as described below. The processors could include one or more processors to control operation of the manipulator 14. The processors can be any type of microprocessor, multi-processor, and/or multi-core processing system. The manipulator controller 26 may additionally, or alternatively, comprise one or more microcontrollers, field programmable gate arrays, systems on a chip, discrete circuitry, and/or other suitable hardware, software, or firmware that can carry out the functions described herein. The term processor is not intended to limit any implementation to a single processor. The manipulator 14 may also comprise a user interface UI with one or more displays and/or input devices (e.g., push buttons, keyboard, mouse, microphone (voice-activation), gesture control devices, touchscreens, etc.).

The tool 20 may comprise a tool controller 21 to control operation of the tool 20, such as to control power to the tool (e.g., to a rotary motor of the tool 20), control movement of the tool 20, control irrigation/aspiration of the tool 20, and/or the like. The tool controller 21 may be in communication with the manipulator controller 26 or other components. The tool 20 may also comprise a user interface UI with one or more displays and/or input devices (e.g., push buttons, keyboard, mouse, microphone (voice-activation), gesture control devices, touchscreens, etc.). The manipulator controller 26 controls a state (position and/or orientation) of the tool 20 (e.g., the tool center point (TCP)) with respect to a coordinate system, such as the manipulator coordinate system MNPL. The manipulator controller 26 can control (linear or angular) velocity, acceleration, or other derivatives of motion of the tool 20.

The TCP, in one example, is a predetermined reference point defined at the energy applicator 24. The geometry of the energy applicator 24 is known in or defined relative to a TCP coordinate system. The TCP may be located at the spherical center of the bur 25 of the tool 20 such that one point is tracked. The TCP may be defined in various ways depending on the configuration of the energy applicator 24. The control of the tool 20 is not limited to a center point. For example, any suitable primitives, meshes, etc., can be used to represent the tool 20.

The shaft 33 of the tool 20 has a known, or able to be calculated (i.e., not necessarily static), pose relative to other coordinate systems. The manipulator 14 could employ the joint/motor encoders, or any other non-encoder position sensing method, to enable a pose of the shaft 33 to be determined. The manipulator 14 may use joint measurements to determine pose of the shaft 33 and/or could employ techniques to measure pose of the shaft 33 directly.

As shown in FIG. 1, the system 10 may further include a navigation system 32. One example of the navigation system 32 is described in U.S. Patent No. 9,008,757, filed on September 24, 2013, entitled "Navigation System Including Optical and Non-Optical Sensors". The navigation system 32 tracks movement of various objects. Such objects include, for example, the manipulator 14, the tool 20 and the anatomy, e.g., femur F and tibia T. The navigation system 32 tracks these objects to gather state information of the objects with respect to a (navigation) localizer coordinate system LCLZ. Coordinates in the localizer coordinate system LCLZ may be transformed to the manipulator coordinate system MNPL, and/or vice-versa, using transformations.

The navigation system 32 includes a cart assembly 34 that houses a navigation controller 36, and/or other types of control units. A navigation user interface UI is in operative communication with the navigation controller 36. The navigation user interface UI includes one or more displays 38. The navigation system 32 can display a graphical representation of the relative states of the tracked objects to the user using the one or more displays 38. The navigation user interface UI further comprises one or more input devices to input information into the navigation controller 36 or otherwise to select/control certain aspects of the navigation controller 36. Such input devices include interactive touchscreen displays. The input devices may include any one or more of push buttons, a keyboard, a mouse, a microphone (voice-activation), gesture control devices, and the like.

The navigation system 32 also includes a navigation localizer 44 coupled to the navigation controller 36. In one example, the localizer 44 is an optical localizer and includes a camera unit 46. The camera unit 46 has an outer casing 48 that houses one or more optical sensors 50. The localizer 44 may comprise its own localizer controller 49 and may further comprise a video camera VC, such as a machine vision camera.

The navigation system 32 includes one or more trackers. In one example, the trackers include a pointer tracker PT, one or more manipulator trackers 52A, 52B, a first patient tracker 54, and a second patient tracker 56. In the illustrated example of FIG. 1, the manipulator tracker is firmly attached to the tool 20 (i.e., tracker 52A), the first patient tracker 54 is firmly affixed to the femur F of the patient 12, and the second patient tracker 56 is firmly affixed to the tibia T of the patient 12. In this example, the patient trackers 54, 56 are firmly affixed to sections of bone. The pointer tracker PT is firmly affixed to a pointer P used for registering the anatomy to the localizer coordinate system LCLZ. The manipulator trackers 52A, 52B may be affixed to any suitable component of the manipulator 14, in addition to, or other than, the tool 20, such as the base 16 (i.e., tracker 52B), or any one or more links 18 of the manipulator 14. The trackers 52A, 52B, 54, 56, PT may be fixed to their respective components in any suitable manner. For example, the trackers may be rigidly fixed, flexibly connected (optical fiber), or not physically connected at all (ultrasound), as long as there is a suitable (supplemental) way to determine the relationship (measurement) of that respective tracker to the object that it is associated with.

Any one or more of the trackers may include active markers 58. The active markers 58 may include light emitting diodes (LEDs). Alternatively, the trackers 52A, 52B, 54, 56, PT may have passive markers, such as reflectors, which reflect light emitted from the camera unit 46. Other suitable markers not specifically described herein may be utilized.

The localizer 44 tracks the trackers 52A, 52B, 54, 56, PT to determine a state of the trackers 52A, 52B, 54, 56, PT, which corresponds respectively to the state of the object respectively attached thereto. The localizer 44 may perform known triangulation techniques to determine the states of the trackers 52A, 52B, 54, 56, PT, and associated objects. The localizer 44 provides the state of the trackers 52A, 52B, 54, 56, PT to the navigation controller 36. In one example, the navigation controller 36 determines and communicates the state of the trackers 52A, 52B, 54, 56, PT to the manipulator controller 26. As used herein, the state of an object includes, but is not limited to, data that defines the position and/or orientation of the tracked object or equivalents/derivatives of the position and/or orientation. For example, the state may be a pose of the object, and may include linear velocity data, and/or angular velocity data, and the like.

The navigation controller 36 may comprise one or more computers, or any other suitable form of controller. The navigation controller 36 has a central processing unit (CPU) and/or other processors, memory (not shown), and storage (not shown). The processors can be any type of processor, microprocessor, or multi-processor system. The navigation controller 36 is loaded with software. The software, for example, converts the signals received from the localizer 44 into data representative of the position and orientation of the objects being tracked. The navigation controller 36 may additionally, or alternatively, comprise one or more microcontrollers, field programmable gate arrays, systems on a chip, discrete circuitry, and/or other suitable hardware, software, or firmware that can carry out the functions described herein. The term processor is not intended to limit to a single processor.

Although one example of the navigation system 32 is shown that employs triangulation techniques to determine object states, the navigation system 32 may have any other suitable configuration for tracking the manipulator 14, tool 20, and/or the patient 12. In another example, the navigation system 32 and/or localizer 44 are ultrasound-based. For example, the navigation system 32 may comprise an ultrasound imaging device coupled to the navigation controller 36. The ultrasound imaging device images any of the aforementioned objects, e.g., the manipulator 14, the tool 20, and/or the patient 12, and generates state signals to the navigation controller 36 based on the ultrasound images. The ultrasound images may be 2-D, 3-D, or a combination of both. The navigation controller 36 may process the images in near real-time to determine states of the objects. The ultrasound imaging device may have any suitable configuration and may be different than the camera unit 46 as shown in FIG. 1.

In another example, the navigation system 32 and/or localizer 44 are radio frequency (RF)-based. For example, the navigation system 32 may comprise an RF transceiver coupled to the navigation controller 36. The manipulator 14, the tool 20, and/or the patient 12 may comprise RF emitters or transponders attached thereto. The RF emitters or transponders may be passive or actively energized. The RF transceiver transmits an RF tracking signal and generates state signals to the navigation controller 36 based on RF signals received from the RF emitters. The navigation controller 36 may analyze the received RF signals to associate relative states thereto. The RF signals may be of any suitable frequency. The RF transceiver may be positioned at any suitable location to track the objects using RF signals effectively. Furthermore, the RF emitters or transponders may have any suitable structural configuration that may be much different than the trackers 52A, 52B, 54, 56, PT shown in FIG. 1.

In yet another example, the navigation system 32 and/or localizer 44 are electromagnetically based. For example, the navigation system 32 may comprise an EM transceiver coupled to the navigation controller 36. The manipulator 14, the tool 20, and/or the patient 12 may comprise EM components attached thereto, such as any suitable magnetic tracker, electro-magnetic tracker, inductive tracker, or the like. The trackers may be passive or actively energized. The EM transceiver generates an EM field and generates states. Such navigation system 32 examples may have structural configurations that are different than the navigation system 32 configuration shown in FIG. 1.

The navigation system 32 may have any other suitable components or structure not specifically recited herein. Furthermore, any of the techniques, methods, and/or components described above with respect to the navigation system 32 shown may be implemented or provided for any of the other examples of the navigation system 32 described herein. For example, the navigation system 32 may utilize solely inertial tracking or any combination of tracking techniques, and may additionally, or alternatively, comprise fiber optic-based tracking, machine-vision tracking, and the like.

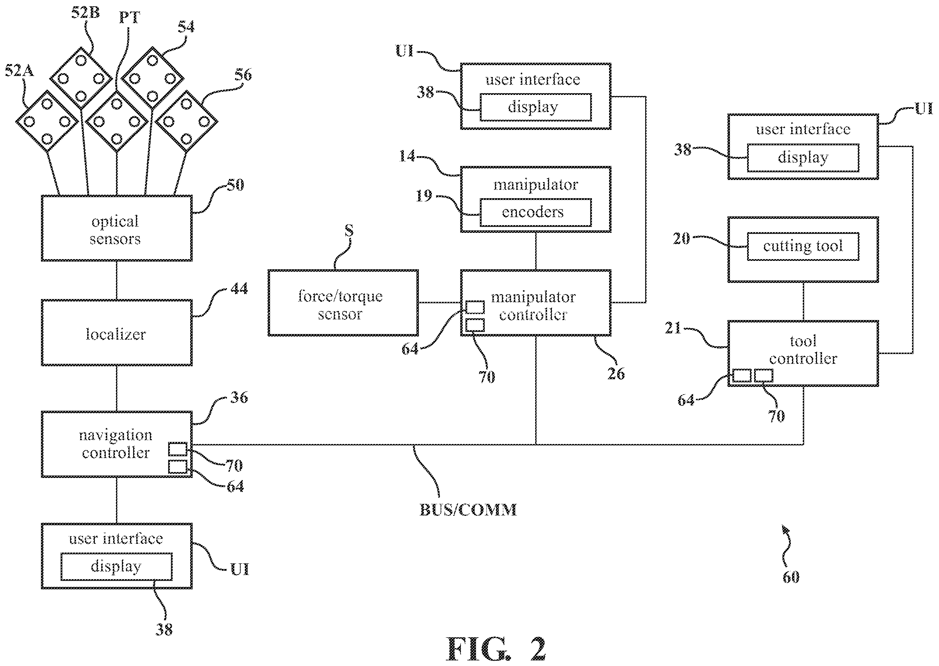

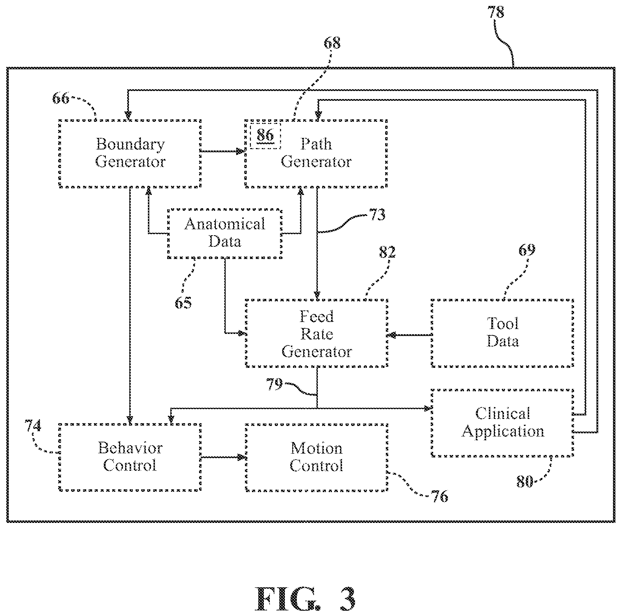

Referring to FIG. 2, the system 10 includes a control system 60 that comprises, among other components, the manipulator controller 26, the navigation controller 36, and the tool controller 21. The control system 60 further includes one or more software programs and software modules shown in FIG. 3. The software modules may be part of the program or programs that operate on the manipulator controller 26, navigation controller 36, tool controller 21, or any combination thereof, to process data to assist with control of the system 10. The software programs and/or modules include computer readable instructions stored in non-transitory memory 64 on the manipulator controller 26, navigation controller 36, tool controller 21, or a combination thereof, to be executed by one or more processors 70 of the controllers 21, 26, 36. The memory 64 may be any suitable configuration of memory, such as RAM, non-volatile memory, etc., and may be implemented locally or from a remote database. Additionally, software modules for prompting and/or communicating with the user may form part of the program or programs and may include instructions stored in memory 64 on the manipulator controller 26, navigation controller 36, tool controller 21, or any combination thereof. The user may interact with any of the input devices of the navigation user interface UI or other user interface UI to communicate with the software modules. The user interface software may run on a separate device from the manipulator controller 26, navigation controller 36, and/or tool controller 21.

The control system 60 may comprise any suitable configuration of input, output, and processing devices suitable for carrying out the functions and methods described herein. The control system 60 may comprise the manipulator controller 26, the navigation controller 36, or the tool controller 21, or any combination thereof, or may comprise one of these controllers. These controllers may communicate via a wired bus or communication network as shown in FIG. 2, via wireless communication, or otherwise. The control system 60 may also be referred to as a controller. The control system 60 may comprise one or more microcontrollers, field programmable gate arrays, systems on a chip, discrete circuitry, sensors, displays, user interfaces, indicators, and/or other suitable hardware, software, or firmware that is capable of carrying out the functions described herein.

Referring to FIG. 3, the software employed by the control system 60 may utilize anatomical data 65. The anatomical data 65 may include imaging data of an anatomical volume AV of the patient 12. The imaging data may be obtained via any suitable imaging modality, such as MRI, X-Ray/Fluoroscopy, CT, and/or Ultrasound. The imaging data may include or be used to derive a geometry of the anatomical volume AV. For example, the imaging data may include imaged slices, such as CT slices which, in combination, form the geometry of the anatomical volume AV. The anatomical data 65 may also include Digital Imaging and Communications in Medicine (DICOM) data of the anatomical volume AV, which may include intercept and slope values of each slice, slice thickness, and a position of the patient 12 at a time of imaging.

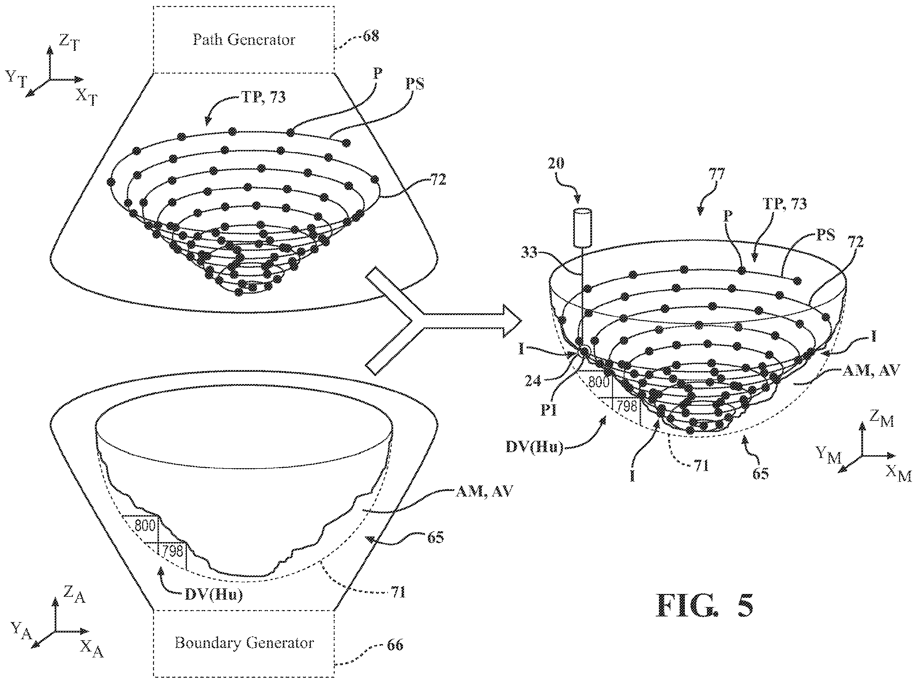

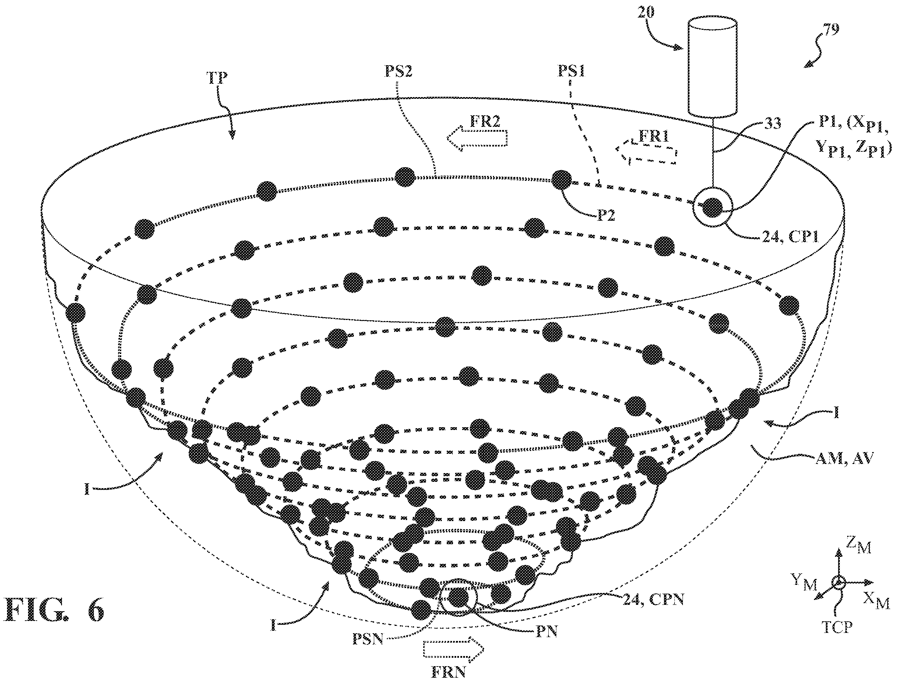

The geometry of the anatomical volume AV may be a 2-D or a 3-D geometry. Referring to FIG. 5, the 3-D geometry is represented using an anatomical model AM. In FIG. 6, the anatomical model AM is a 3-D bone model of an acetabulum AM of the patient 12. As described, the bone model could be of any other type of bone, such as a femur, tibia, scapula (glenoid), humerus, vertebra, or the like. The anatomical model AM may be formed of imaged slices, such as CT slices, of the anatomical volume AV. Additionally, the 3-D bone model may be a patient-specific bone model (e.g., based on a bone of the patient 12) or a statistical bone model (e.g., based on a bone of a representative patient). The bone model can be formed of any suitable method, such as manual or automated segmentation of imaging data, morphing of the statistical bone model, or the like.

The anatomical data 65 may also include density values DV of an anatomical volume AV. For instance, referring to FIG. 5, the anatomical data 65 may include radiodensity values DV that are associated with the anatomical model AM. The radiodensity values DV may be mapped to coordinates, pixels, and/or voxels of the anatomical model AM. In this way, the anatomical data 65, in addition to providing the anatomical model AM, also provides density values DV that correspond to the anatomical model AM. The density values DV may be any suitable density value. For instance, the density values DV may be measured in Hounsfield units (HU), as shown in FIG. 5. The density values DV can be determined or derived using any suitable technique. For example, the density values DV could be detected from electrical conductivity measurements of the anatomical volume AV. As another example, the density values DV may be determined using a density phantom that is imaged simultaneously with the anatomy. The density phantom may be imaged using bone densitometry, absorptiometry, quantitative computerized tomography, ultrasonography, dual-energy X-ray absorptiometry (DEXA), scanning electron microscopy (SEM), or any other type of image analysis, or the like. The density values DV can be included in the DICOM data. Density values could also be obtained intra-operatively using, e.g., a CT scanning device in the operating room.

The control system 60 may be configured to normalize the density values DV. In some instances, the density values DV may vary depending on, among other things, the imaging modality and/or the system used to image the anatomical volume AV. For example, the density values DV may vary if the anatomical volume AV is scanned using an MRI or a CT. As another example, the density values DV may vary based on a type or brand of scanner used to image the anatomical volume AV. In such instances the control system 60 may normalize the density values DV to provide consistent interpretation or evaluation of the density values DV for the techniques described herein. In one example, the control system 60 may normalize or calibrate the density values DV based on parameters of a density phantom that is imaged with the anatomical volume AV. In some instances, the control system 60 may normalize the density values DV by controlling the tool 20 to interact with the anatomical volume AV and measuring forces and torques related the interaction. For example, the control system 60 may control the tool 20 to cut a tissue of the anatomical volume AV and measure forces placed on the tool 20 (e.g., a motor 27 of the tool 20). The density values DV can be normalized by the control system 60 comparing the measured forces on the tool 20 to the raw density values.

Referring to FIG. 3, the software employed by the control system 60 can include a boundary generator 66. As shown in FIG. 6, the boundary generator 66 is a software program or module that generates a virtual object 71 for constraining movement and/or operation of the tool 20. The virtual object 71 may be a virtual boundary and can be one-dimensional, two-dimensional, or three-dimensional, and may comprise a point, line, axis, trajectory, plane, or other shapes, including complex geometric shapes. In some embodiments, the virtual object 71 is a surface defined by a polygonal or triangular mesh. The virtual objects 71 may be defined with respect to the anatomical model AM and can define a region of the anatomical model AM for which material is allowed to be removed by the tool 20 from a region of the anatomical model AM for which material is prohibited from being removed by the tool 20. In the example of FIG. 5, the virtual objects 71 are 3-D boundaries that delineate an area for an acetabular component of a hip replacement surgery and are associated with the 3-D anatomical model AM of the acetabulum. The anatomical model AM is registered to the one or more patient trackers 54, 56 such that the virtual objects 71 become associated with the anatomical model AM. The virtual objects 71 may be implant-specific, e.g., defined based on a size, shape, volume, etc. of an implant and/or patient-specific, e.g., defined based on the patient's anatomy. The virtual objects 71 may be created pre-operatively, intra-operatively, or combinations thereof. In other words, the virtual objects 71 may be defined before the surgical procedure begins, during the surgical procedure (including during tissue removal), or combinations thereof. In any case, the control system 60 obtains the virtual objects 71 by storing/retrieving the virtual objects 71 in/from memory, obtaining the virtual objects 71 from memory, creating the virtual objects 71 pre-operatively, creating the virtual objects 71 intra-operatively, or the like.

The manipulator controller 26 and/or the navigation controller 36 track the state of the tool 20 relative to the virtual objects 71. In one example, the state of the TCP is measured relative to the virtual objects 71 for purposes of determining forces to be applied to a virtual rigid body model via a virtual simulation. This determination can be made, in one example, so that the tool 20 remains in a desired positional relationship to the virtual objects 71 (e.g., not moved beyond them). The results of the virtual simulation are commanded to the manipulator 14. The control system 60 controls/positions the manipulator 14 in a manner that emulates the way a physical handpiece would respond in the presence of physical boundaries/barriers. The boundary generator 66 may be implemented on the manipulator controller 26. Alternatively, the boundary generator 66 may be implemented on other components, such as the navigation controller 36.

Referring to FIG. 3, a path generator 68 can be another software program or module run by the control system 60. In one example, the path generator 68 is run by the manipulator controller 26 and generates path data 73. The path data 73 may include a tool path TP along which the manipulator 14 moves a tool 20 to interact with the anatomical volume AV. For example, the tool 20 may traverse the tool path TP to remove sections of the anatomical volume AV to prepare the anatomical volume AV for receiving an implant. The tool path TP may comprise a plurality of path segments PS, or may comprise a single path segment PS. The path segments PS may be straight segments, curved segments, combinations thereof, or the like. The tool path TP may also be defined with respect to the anatomical model AM. The tool path TP may be predetermined based on a geometry of an implant model selected for the anatomical model AM and/or a planned resection volume of the anatomical model AM. In other words, the tool path TP may be implant-specific, e.g., defined based on a size, shape, volume, etc. of an implant, and/or patient-specific, e.g., defined based on the patient's anatomy. In some instances, the path data 73 may include a set of potential tool paths TP, which are evaluated to determine which tool path TP optimizes cutting performance.

In some instances, the path data 73 may also include a predefined pose of the shaft 33 of the tool 20. For example, the predefined pose of the shaft 33 may include a state (e.g., a position and/or orientation) of the shaft 33 with respect to the manipulator coordinate system MNPL. As previously stated, the manipulator 14 could employ the joint/motor encoders, or any other non-encoder position sensing method, to enable a pose of the shaft 33 to be determined. As such, the predefined pose may also be provided in terms of a sensed reading of the joint/motor encoders, or any other non-encoder position sensing method. Therefore, the path data 73 may include not only the tool path TP, but may also include predefined poses of the shaft 33 along the tool path TP. For instance, the path data 73 may include a predefined pose of the shaft 33 corresponding to a path segment PS of the tool path TP.

In one version described herein, the tool path TP is defined as a tissue removal path, but, in other versions, the tool path TP may be used for treatment other than tissue removal. One example of the tissue removal path described herein comprises a milling path 72. It should be understood that the term "milling path" generally refers to the path of the tool 20 in the vicinity of the target site for milling the anatomy and is not intended to require that the tool 20 be operably milling the anatomy throughout the entire duration of the path. For instance, the milling path 72 may comprise sections or segments where the tool 20 transitions from one location to another without milling. Additionally, other forms of tissue removal along the milling path 72 may be employed, such as tissue ablation, and the like. The milling path 72 may be a predefined path that is created pre-operatively, intra-operatively, or combinations thereof. In other words, the milling path 72 may be defined before the surgical procedure begins, during the surgical procedure (including during tissue removal), or combinations thereof. In any case, the control system 60 obtains the milling path 72 by storing/retrieving the milling path 72 in/from memory, obtaining the milling path 72 from memory, creating the milling path 72 pre-operatively, creating the milling path 72 intra-operatively, or the like. The milling path 72 may have any suitable shape, or combinations of shapes, such as circular, helical/corkscrew, linear, curvilinear, combinations thereof, and the like.

One example of a system and method for generating the virtual objects 71 and/or the milling path 72 is described in U.S. Patent No. 9,119,655, entitled "Surgical Manipulator Capable of Controlling a Surgical Instrument in Multiple Modes". In some examples, the virtual objects 71 and/or milling paths 72 may be generated offline rather than on the manipulator controller 26 or navigation controller 36. Thereafter, the virtual objects 71 and/or milling paths 72 may be utilized at runtime by the manipulator controller 26.

A path interpolator 86, as shown in FIG. 3, can be a sub-module of the path generator 68. Path interpolator 86 determines target positions for a coordinate system of the energy applicator 24 along the tool path TP. The pose of the shaft 33 is fixed relative to the coordinate system of the energy applicator 24. Referring to FIG. 5, these target positions are points P along the tool path TP which the energy applicator 24 will successively pass to perform the task. In one implementation, the path segments PS of the tool path TP are defined between subsequent points P. Inputs into the path interpolator 86 can include but are not limited to: the data defining the origin and terminus of a path segment PS; the data indicating if the path segment PS is straight or curved and, if curved, the characteristics of the curve. Another input into the path interpolator 86 is a feed rate, which will be described in greater detail in the subsequent section. This is a predetermined rate (speed or velocity) at which the energy applicator 24 is set to travel along the path segment PS. Based on the above input variables, the path interpolator 86, in one implementation, can determine the target positions of the energy applicator 24 according to the following steps: 1) The origin of the coordinate system of energy applicator 24 is assumed to be at an initial position. The initial position is a position along the path segment PS over which the energy applicator 24 should travel. If the energy applicator 24 is at the beginning point P of the path segment PS, this point P is the initial position of the coordinate system of energy applicator 24. Both the initial position and the target position are points P in the bone coordinate system. 2) Based on the feed rate, the distance along which the energy applicator 24 would travel along the path segment PS in a single time frame is calculated. In some versions, the period of a time frame is 0.1 to 2 milliseconds. 3) Based on the initial position, the length of the calculated distance and the location of the segment terminus, the path interpolator 86 generates data defining the target position. A further variable that can be used to determine target positions are data from the tool path generator describing the characteristics of the path segment PS: straight or curved; and, if curved, the radius of curvature. 4) Steps 1 through 3 are repeated until it is determined that the coordinate system has reached the terminus of the path segment. After the calculation of the first target position spaced from the segment origin, the target position calculated in each frame is employed as the initial position upon which the calculation of the next frame's target position is based. 5) Once the target position equals the terminus position for a path segment PS, the path interpolator 86 repeats steps 1 through 4 to generate a set of target positions that are located along the new segment. During the time period of a single frame, the distance the energy applicator 24 can travel may be greater than the distance to the terminus position for the current segment. If the path interpolator 86 determines that the energy applicator 24 would be in this state, the path interpolator 86, for a time point starting when it is determined that the energy applicator 24 would be at the terminus of the current path segment PS, generates data indicating where the energy applicator 24 should be located at along the next path segment PS at the end of that frame. The techniques described herein are not exclusively limited to this method for determining target positions.

Referring to FIG. 3, the software employed by the control system 60 may further utilize tool data 69. The tool data 69 includes geometry of the tool 20 that will interact with the tool path TP. For example, the tool data 69 may include a 3-D geometry, such as a 3-D model of the energy applicator 24 of the tool 20, to specify a shape of a cutting surface of the tool 20. For instance, the 3-D geometry may specify that the cutting surface of the energy applicator 24 of the tool 20 includes a circular cross-sectional area, a triangular cross-sectional area, a flat cross-sectional area, or a tapered cross-sectional area. The 3-D geometry may include slices of the energy applicator 24 extracted from the 3-D model or any other source. In some instances, the tool data 69 may include a shape of the cutting surface, size, surface area, volume, and/or dimensions of the energy applicator 24. The tool data 69 may also include a preferred use of the tool 20. For example, in some instances, the cutting surface of the energy applicator 24 may be positioned at a side of the energy applicator 24 to promote cutting using sides of the energy applicator 24, instead of a tip of the energy applicator 24. The tool data 69 may also include a preferred cutting depth corresponding to a particular tool 20. For example, a particular tool 20 may be used to perform a very shallower cut, whereas another tool 20 may be used to perform a deeper cut. The tool data 69 may also include a shaft 33 of the tool 20. The tool data 69 can be obtained from tool parameters, specifications, or calibration data and/or pre-operatively measured.

Referring to FIG. 3, another software program or module run by the control system 60 may be a behavior controller 74. Behavior control is the process of computing data that indicates the next commanded position CP of the energy applicator 24 and/or orientation (e.g., pose) for the shaft 33 of the tool 20. In some cases, the position of the TCP is output from the behavior controller 74, while in other cases, the position and orientation of the tool 20 is output. Output from the boundary generator 66, the path generator 68, and a force/torque sensor S may feed as inputs into the behavior controller 74 to determine the next commanded position CP for the energy applicator 24 and/or orientation for the tool 20. The behavior controller 74 may process these inputs, along with one or more virtual constraints, to determine the commanded pose.

Referring to FIG. 3, yet another software program or module run by the control system 60 may be a motion controller 76. One aspect of motion control is the control of the manipulator 14. The motion controller 76 receives data defining the next commanded pose from the behavior controller 74. Based on these data, the motion controller 76 can determine the next position of the joint angles of the joints J of the manipulator 14 (e.g., via inverse kinematics and Jacobian calculators) so that the manipulator 14 is able to position the tool 20 as commanded by the behavior controller 74, e.g., at the commanded pose. In other words, the motion controller 76 processes the commanded pose, which may be defined in Cartesian space, into joint angles of the manipulator 14, so that the manipulator controller 26 can command the joint motors 27 accordingly, to move the joints J of the manipulator 14 to commanded joint angles corresponding to the commanded pose of the tool 20. In one version, the motion controller 76 regulates the joint angle of the joints J and adjusts the torque that joint motors 27 output to, as closely as possible, ensure that the joint motor 27 drives the associated joint J to the commanded joint angle.

Any of the boundary generator 66, path generator 68, behavior controller 74, motion controller 76, and feed rate generator 67 (to be described later) may be sub-sets of a software program 78 or software programs that operate separately and/or independently in any combination thereof. The term "software program" is used herein to describe the computer-executable instructions that are configured to carry out the various capabilities of the technical solutions described. For simplicity, the term "software program" is intended to encompass, at least, any one or more of the boundary generator 66, path generator 68, behavior controller 74, motion controller 76, and/or feed rate generator 82. The software program 78 can be implemented on the manipulator controller 26, navigation controller 36, or any combination thereof, or may be implemented in any suitable manner by the control system 60.

A clinical application 80 may be provided to handle user interaction. The clinical application 80 can handle many aspects of user interaction and coordinates the surgical workflow, including pre-operative planning, implant placement, registration, bone preparation visualization, feed-rate parameter adjustment or confirmation, and post-operative evaluation of implant fit, etc. The clinical application 80 is configured to output to one or more of the displays 38. The clinical application 80 may run on its own separate processor or may run alongside the navigation controller 36. In one example, the clinical application 80 interfaces with the boundary generator 66 and/or path generator 68 after implant placement is set by the user, and then sends the virtual object 71 and/or tool path TP returned by the boundary generator 66 and/or path generator 68 to the manipulator controller 26 for execution.

The system 10 may operate in a force-input or manual mode, such as described in U.S. Patent No. 9,119,655. Here, the user applies a force to the manipulator 14, and in response, the manipulator 14 executes movement of the tool 20 and its energy applicator 24 at the surgical site. In one example, the user physically contacts the tool 20 to cause movement of the tool 20 in the manual mode. In one version, the manipulator 14 monitors forces and torques placed on the tool 20 by the user in order to position the tool 20. For example, the manipulator 14 may comprise the force/torque sensor S that detects the forces and torques applied by the user and generates corresponding input used by the control system 60 (e.g., one or more corresponding input/output signals).

The manipulator controller 26 and/or the navigation controller 36 receives the input (e.g., signals) from the force/torque sensor S. In response to the user-applied forces and torques, the manipulator 14 moves the tool 20 in a manner that emulates the movement that would have occurred based on the forces and torques applied by the user. Movement of the tool 20 in the manual mode may also be constrained in relation to the virtual objects 71 generated by the boundary generator 66. In some versions, measurements taken by the force/torque sensor S are transformed from a force/torque coordinate system FT of the force/torque sensor S to another coordinate system, such as a virtual mass coordinate system in which a virtual simulation is carried out on the virtual rigid body model of the tool 20 so that the forces and torques can be virtually applied to the virtual rigid body in the virtual simulation to ultimately determine how those forces and torques (among other inputs) would affect movement of the virtual rigid body.

The system 10 may also operate in a semi-autonomous mode in which the manipulator 14 moves the tool 20 along the tool path TP in an automated manner. An example of operation in the semi-autonomous mode is also described in U.S. Patent No. 9,119,655. In some embodiments, when the manipulator 14 operates in the semi-autonomous mode, the manipulator 14 can move the tool 20 free of user applied force such that a user does not physically contact the tool 20 to move the tool 20. Instead, the user may use some form of remote control or switch to control starting and stopping of automated movement. For example, the user may hold down a button of the remote control to start movement of the tool 20 and release the button to stop movement of the tool 20, as also described in U.S. Patent No. 9,119,655.

### II. Determining the Feed Rate

The speed or velocity at which the energy applicator 24 advances is referred to as the feed rate. In one implementation, the energy applicator 24 may advance along the tool path TP at one or more feed rates. More specifically, such advancement can occur during operation of the manipulator 14 in the semi-autonomous mode. In one implementation, this section describes techniques in which the feed rate is determined based on an interaction between the tool path TP and the anatomical model AM in order to generate cut plan data 79 (shown in FIG. 6 and explained herein). The feed rate computation techniques described herein can be augmented by the feed rate techniques described in U.S. Patent No. 9,119,655.

Referring to FIG. 3, the software employed by the control system 60 can include a feed rate generator 82. The feed rate generator 82 determines the feed rate, or feed rate factors, at which the energy applicator 24 should move as it travels along one or more individual path segments PS of the tool path TP (see FIG. 5) to optimize cutting performance. The feed rate factor can be a scalar value. In practice, the manipulator controller 26 may be provided with plural feed rate factors. These feed rate factors can be used to set default feed rates and/or can be scaled or modified according to variables, as described below. The feed rate factors need not always correspond to the actual feed rate by which the energy applicator 24 is advanced.

The feed rate generator 82 can determine the feed rate(s) or feed rate factors and associate such with the tool path TP. The feed rate generator 82 can do so pre-operatively and/or intraoperatively. In some examples, the feed rate generator 82 does so pre-operatively, and the surgeon can modify or adjust the output of the feed rate generator 82 at a later time, using, e.g., the clinical application 80.

The generator 82 is not intended to be limited exclusively to feed rate. To the extent that tool operating parameters other than feed rate are generated, the generator 82 can be identified using any suitable terminology, such as tool parameter generator 82, tool operating parameter generator 82, and the like.

### i. Cut Plan Data Overview

The control system 60, using the feed rate generator 82 can generate or output cut plan data 79. The cut plan data 79 can be understood as the planned tool path TP, path segments PS, and optionally, planned feed rates or feed rate factors for the tool that are associated with the tool path TP or path segments. An example illustration of the cut plan data 79 is shown in FIG. 6. As shown, the cut plan data 79 includes a planned feed rate factor FR1...FRN assigned to each path segment PS1...PSN of the tool path TP. The feed rate generator 82 is configured to determine the planned feed rate factor FR1...FRN assigned to each path segment PS1...PSN based on simulating an interaction between the anatomical model AM and a tool 20 at a point P1...PN. The cut plan data 79 can include any other parameters (such as tool operating parameters) that relate to how the tool and/or manipulator 14 can be controlled to cut the anatomy. These parameters can include, but are not limited to, virtual object 71, stiffness/damping parameters of the tool or mesh, cutting speed, cutting depth, number of tool passes or tool segments, cutting force, tissue temperature, and the like.

One example of the cut plan data 79 and tool path TP are illustrated in FIG. 6 with respect to an example involving an acetabulum. The cut plan data 79 and the tool path TP may vary from what is illustrated. For example, the tool path TP may include a greater or lesser number of points P and path segments PS. As another example, the cut plan data 79 may include a greater or lesser number of planned feed rate factors FR1...FRN. The tool path TP may also be different from what is shown depending on the region of the anatomy or implant.

Referring to FIG. 6, the one or more controllers, including but not limited to the manipulator controller 26, including the path generator 68 and feed rate generator 82, are configured to control the manipulator 14 to advance the energy applicator 24 of the tool 20 to a plurality of commanded positions CP1-CPN according to the cut plan data 79. The commanded positions CP are often, but not necessarily, defined with respect to points P along the tool path TP in the semi-autonomous mode. Alternatively, the commanded positions CP may be determined pursuant to user-initiated motion that is emulated by the system 10 in the manual mode.

### ii. Method of Generating Cut Plan Data

In this subsection, a method of generating cut plan data 79 based on simulating an interaction between the tool path TP and the anatomical model AM will be described.

### a. Obtaining the Anatomical Data, the Path Data, and the Tool Data

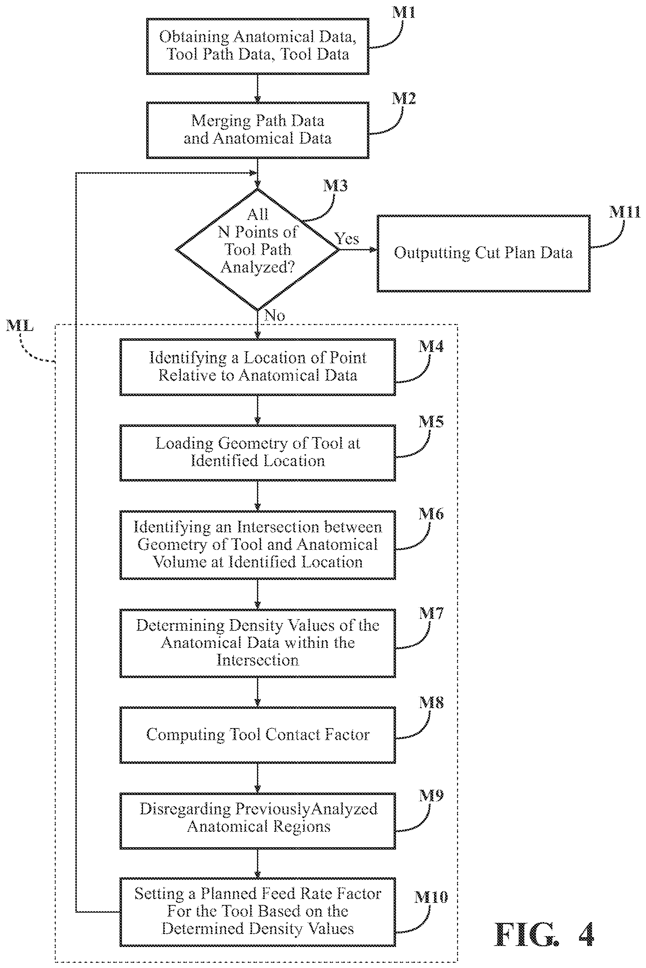

An example of the method for generating cut plan data 79 is shown in FIG. 4. In this implementation, the method for generating cut plan data 79 includes a step M1 of obtaining the anatomical data 65, the path data 73, and the tool data 69. This information is obtained by the control system 60, and more specifically, the feed rate generator 82. Referring to FIG. 3, the feed rate generator 82 is shown as being in communication with the path generator 68 (for receiving the path data 73), as well as a source (e.g., memory) that includes the anatomical data 65 and tool data 69. The anatomical data 65, the path data 73, and the tool data 69 serve as inputs to the feed rate generator 82.

### b. Merging the Anatomical Data and the Path Data

Referring to FIG. 4, the method includes a step M2 of merging the path data 73 and the anatomical data 65. After obtaining the anatomical data 65 and the path data 73 during step M1, the control system 60, or feed rate generator 82, processes the data by merging the data into a common coordinate system. The step M2 of merging the path data 73 and the anatomical data 65 is further shown in one illustrative example in FIG. 5. As shown, the path data 73 includes the tool path TP and points P along the tool path TP, the tool path TP and the points P being mapped on a path data coordinate system X_{T}, Y_{T}, Z_{T}. The anatomical data 65 includes the anatomical model AM and density values DV, which are mapped on an anatomical data coordinate system X_{A}, Y_{A}, Z_{A}. In one example, the path data 73 and the anatomical data 65 are merged such that the tool path TP and the anatomical model AM create a merged model 77, which is mapped on a merged coordinate system X_{M}, Y_{M}, Z_{M}.

The merged coordinate system X_{M}, Y_{M}, Z_{M} may be the anatomical data coordinate system X_{A}, Y_{A}, Z_{A}, the path data coordinate system X_{T}, Y_{T}, Z_{T}, or a new, different, or arbitrary coordinate system. In some instances, the anatomical data 65 may be transformed and superimposed onto the path data 73 such that the path data coordinate system X_{T}, Y_{T}, Z_{T} serves as the merged coordinate system X_{M}, Y_{M}, Z_{M}. For example, the anatomical model AM may be scaled and superimposed onto the path data 73 such that the anatomical model AM is mapped to the path data coordinate system X_{T}, Y_{T}, Z₁. Furthermore, the density values DV may be mapped from coordinates in the anatomical data coordinate system X_{A}, Y_{A}, Z_{A} to coordinates in the path data coordinate system X_{T}, Y_{T}, Z_{T}. In other instances, the path data 73 may be transformed and superimposed onto the anatomical data 65 such that the anatomical data coordinate system X_{A}, Y_{A}, Z_{A}, serves as the merged coordinate system X_{M}, Y_{M}, Z_{M}. For example, the tool path TP may be scaled and superimposed onto the anatomical data 65 such that the tool path TP is mapped to the anatomical data coordinate system X_{A}, Y_{A}, Z_{A}. Furthermore, points P along the tool TP may be mapped from the path data coordinate system X_{T}, Y_{T}, Z_{T} to the anatomical data coordinate system X_{A}, Y_{A}, Z_{A}. In still other instances, the path data 73 and the anatomical data 65 may both be transformed and superimposed onto one another such that the merged coordinate system X_{M}, Y_{M}, Z_{M} is a new coordinate system. For example, the tool path TP and/or the anatomical model AM may be scaled using different factors such that the tool path TP and the anatomical model AM both may be mapped to a new, merged coordinate system X_{M}, Y_{M}, Z_{M}.

This merger can, but need not be, provided or performed in visual form. For instance, the merger can be purely computational and unseen to a user. Alternatively, aspects of these steps can be visualized for the user, using e.g., the clinical application 80 so that the user can experience, or interact with the path data 73 and the anatomical data 65.

### c. Generating the Cut Plan Data

Once the feed rate generator 82 merges the path data 73 and the anatomical data 65, the feed rate generator 82 carries out a series of additional steps to generate the cut plan data 79. These steps will be described in this section. The feed rate generator 82 does so by evaluating or simulating an interaction between the tool 20 and the anatomical model AM at individual point(s) P. For example, referring to FIG. 6, the feed rate generator 82 generates the cut plan data 79 for the tool path TP relative to path segment(s) PS1...PSN by simulating the interaction between the tool 20 and the anatomical model AM at a corresponding point P1...PN. For instance, to generate the cut plan data 79 for path segment PS1, the feed rate generator 82 simulates the interaction between the tool 20 and the anatomical model AM at point P1. For simplicity, cut plan data 79 generated by the feed rate generator 82 for a path segment PS will be referred to herein as cut plan data 79 for a point P. The cut plan data 79 can be the output which includes data related to any number of, or all, path segments PS or points P of the tool path TP.

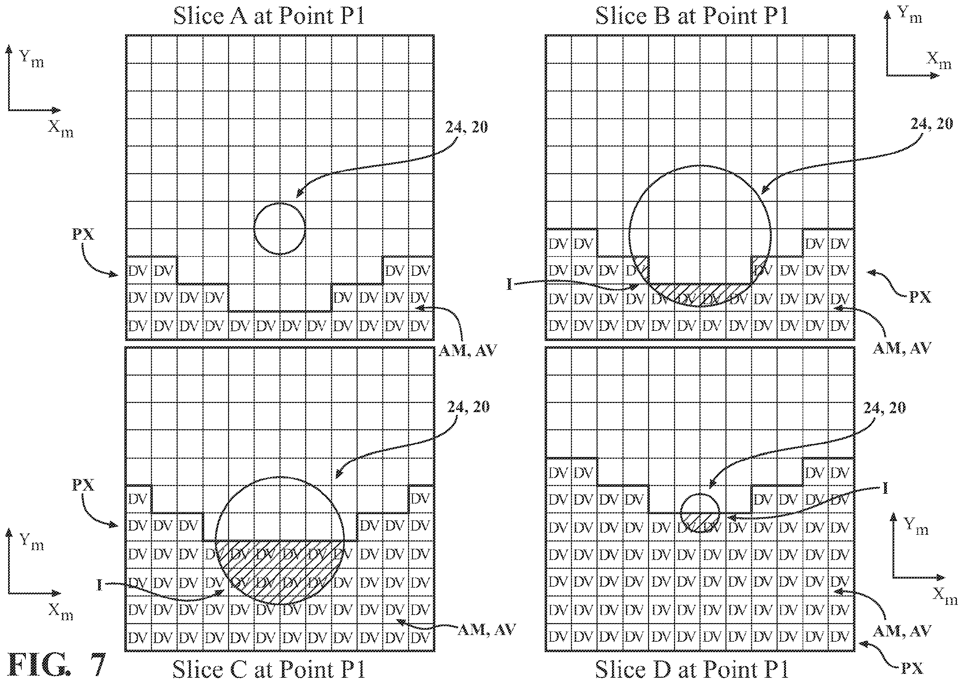

In one instance, the simulated interaction between the tool 20 and the anatomical model AM may include a simulated intersection I between the tool 20 and the anatomical model AM. The feed rate generator 82 may simulate an interaction between the tool 20 and the anatomical model AM and determine whether the tool 20 intersects the anatomical model AM during the simulated interaction. The feed rate generator 82 may then generate the cut plan data 79 based on analyzing the simulated intersection I between the tool 20 and the anatomical model AM. Examples of such intersections I are shown in FIGS. 5-7. Referring to the merged model 77 of FIG. 5, the tool 20 may intersect the anatomical model AM at various example intersections I as the tool 20 travels along the tool path TP. One such intersection I occurs at point PI. Various example intersections I are also shown in FIG. 6 and shown in cross-section in FIG. 7. While these figures illustrate example intersections I they are not intended to limit the scope of the concept. In other instances, the tool path TP may intersect the anatomical model AM at different locations of the anatomical model AM and may intersect the anatomical model AM at a different number of locations of the anatomical model AM. Details related to the intersection I will be described in further below.

To generate the cut plan data 79 for points P along the tool path TP, the method of FIG. 4 can execute a loop ML. In the instance of FIG. 4, the loop ML iterates to generate the cut plan data 79 for N number of points P (and corresponding path segments PS) along the tool path TP in order to generate the cut plan data 79 for the tool path TP. The number of points P can be automatically determined by the program or selected by a user. The method includes a step M3 of determining whether each of the N points P of the tool path TP has been analyzed. At step M3, N can be set to any number of points. In some instances, the feed rate generator 82 may iterate through the loop ML for some, but not all, points P of the tool path TP. For example, in some instances, the feed rate generator 82 may iterate through at least one point P along the tool path TP, one or more grouping of points P along the tool path TP, every point P along the tool path TP that is a multiple of n (n being a number greater than 0), randomly selected points P along the tool path TP, or selected/predetermined points P along the tool path TP. However, in the example shown in FIG. 4, this loop ML evaluates whether all points P of the tool path TP have been analyzed. If the feed rate generator 82 determines that each point P along the tool path TP has been evaluated, the feed rate generator 82 proceeds to step M11 and outputs the cut plan data 79 for the tool path TP. However, if the feed rate generator 82 determines that cut plan data 79 has not been generated for each point P along the tool path TP, the feed rate generator 82 proceeds to step M4 and iterates through the loop ML once more to generate cut plan data 79 for a successive (or remaining) point P along the tool path TP.

As shown, the loop ML includes steps M4-M10 for generating the cut plan data 79 based on the N number of points P along the tool path TP. The steps M4-M10 are evaluated for individual points P. However, the order of these steps M4-M10 need not be executed in the exact order shown, unless a subsequent step relies on a previous step. The loop ML includes a step M4 of identifying a location of a point P on the tool path TP relative to the anatomical data 65; a step M5 of loading, from the tool data 69, the geometry of the tool 20 at the identified location; a step M6 of identifying an intersection between the geometry of the tool 20 and the anatomical volume AV; a step M7 of determining density values DV of the anatomical data 65 within the intersection; a step M8 of computing an intersection ratio related to the geometry of the tool 20 within the intersection relative to the geometry of the tool 20 outside of the intersection; and a step M10 of setting a planned feed rate factor FR for the tool 20 at a path segment PS corresponding to the point P based on the determined density values DV and the computed intersection ratio. The step M9 of disregarding previously determined density values DV will be described herein in a separate subsection.

During step M4, the feed rate generator 82 identifies a location of the point P. Specifically, the feed rate generator 82 identifies the location of the point P relative to the anatomical data 65. Referring to one example in FIG. 6, the feed rate generator 82 may determine that point P1 is located at coordinates (x_{P1}, y_{P1}, z_{P1}) in the merged coordinate system X_{M}, Y_{M}, Z_{M}. The feed rate generator 82 can identify a location of the point P using different methods or using coordinates from any coordinate system.

During step M5, the feed rate generator 82 determines a geometry of the tool 20 at the point P. Specifically, the feed rate generator 82 loads, from the tool data 69, the geometry of the tool 20 at the point P. As previously stated, the cut plan data 79 considers an intersection I between the geometry of the tool 20 and the anatomical volume AV at the point P. To identify the intersection I between the geometry of the tool 20 and the anatomical volume AV at the point P, the feed rate generator 82 loads or provides a geometry of the tool 20 at the point P. For example, the feed rate generator 82 may load a 3-D geometry, such as a 3-D model, of the energy applicator 24 during step M5. The 3-D geometry may include a 3-D volume or slices of the energy applicator 24. The feed rate generator 82 may also load dimensions of the tool 20 (e.g., a radius, diameter, volume, a shape of the cutting surface, or surface area of the energy applicator 24) to determine the geometry of the tool 20.

The feed rate generator 82 may determine the planned feed rate FR based on the tool data 69. For example, the feed rate generator 82 may determine the planned feed rate FR based on the geometry of the tool 20. For instance, the cutting surface of the energy applicator 24 may include a tapered cross-sectional area. In such an instance, the feed rate generator 82 may determine the planned feed rate FR to move the tool 20 at a speed to optimize cutting with the tapered cross-sectional area. As another example, the feed rate generator 82 may determine the planned feed rate FR based on a preferred use of the tool 20. For instance, the cutting surface of the energy applicator 24 may be positioned at a side of the energy applicator 24 to promote cutting using sides of the energy applicator 24 with a swift brushing motion. In such an instance, the feed rate generator 82 may determine the planned feed rate FR to move the tool 20 at a speed to perform a swift brushing motion with the tool 20. As yet another example, the feed rate generator 82 may determine the planned feed rate FR based on a preferred cutting depth of the tool 20. For instance, the tool 20 may be preferably used for shallower cuts. In such an instance, the feed rate generator 82 may determine the planned feed rate FR to move the tool 20 to perform a shallower cut. These planned feed rate FR considerations related to the tool data 69 may be accounted for during step M5 of the method or may be accounted for during any other suitable step of the method.

At step M6 and as shown in one example in FIG. 7, the feed rate generator 82 identifies the intersection I between the geometry of the tool 20 and the anatomical volume AV at a point P1. In this example, the energy applicator 24 is a spherical cutting bur. The geometry that is compared to the anatomical volume AV is a circular cross-section of the energy applicator 24. Accordingly, the size of the circular cross section and the geometry of the anatomical volume AV will differ from slice to slice. At point P1, for any given slice, some portions of the tool geometry may be included within the intersection I, while other parts of the tool 20 may not be included within the intersection I. To identify the intersection I, the feed rate generator 82 can analyze or sweep through imaged slices of the anatomical volume AV and the tool 20 to identify intersections I.

In the instance of FIG. 7, the feed rate generator 82 analyzes four imaged slices A, B, C, D relative to corresponding slices of the tool 20. The slices A, B, C, D are shown for the purposes of illustration. In other instances, the feed rate generator 82 may analyze any suitable number of slices of the anatomical volume AV. Furthermore, the slices may feature a different view of the anatomical volume AV than shown in FIG. 7. Additionally, the geometry of the tool 20 can be different from what is specifically shown.

In this example, the feed rate generator 82 may identify the CT slices for which there is a cross-sectional intersection I between the 3-D geometry of the tool and the anatomical volume AV. Referring to FIG. 7, the cross-sectional intersection I is indicated using a shaded area. As follows, the feed rate generator 82 may identify that at point P1, slices B, C, and D, include the intersection I, while slice A does not include the intersection I.

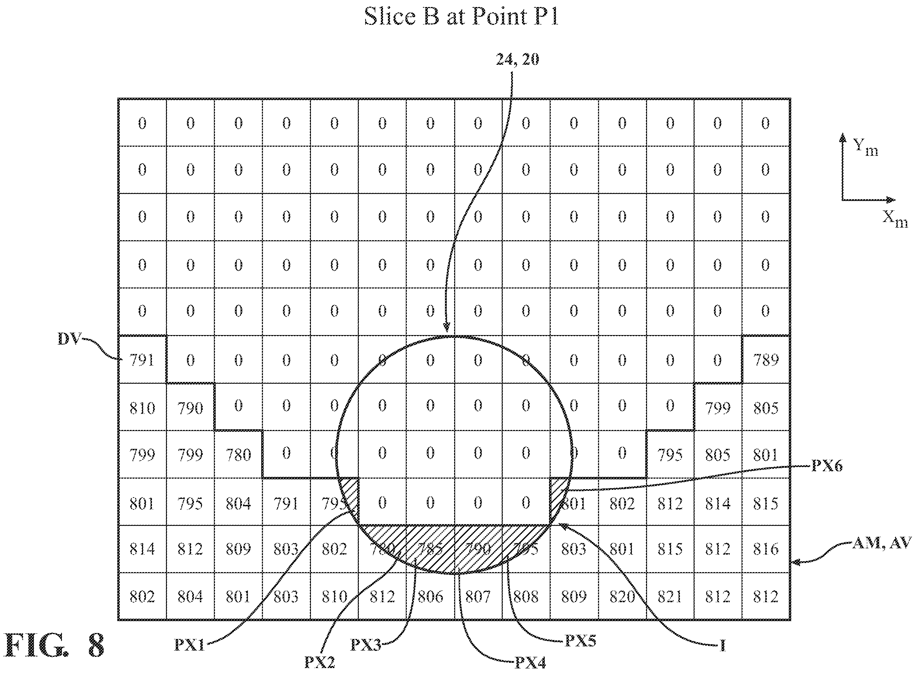

The feed rate generator 82 may identify the intersection I by identifying, for each slice A, B, C, D, a quantity of pixels PX within the intersection I. Referring to FIG. 7, the slices A, B, C, D each include pixels PX. FIG. 8 provides a more detailed view of slice B, where pixels PX are labeled. For slice B, the feed rate generator 82 may identify that six pixels, pixels PX1-PX6, are located within the cross-sectional intersection I. In some instances, the feed rate generator 82 may alternatively calculate an area of the cross-sectional intersection I, in terms of pixels PX (i.e. the area may be based on a size of a single pixel PX).

The pixels PX shown in FIG. 7 (and FIG. 8, to be described herein) are shown for illustrative purposes. In other instances, the pixels PX may be larger or smaller in size. Furthermore, the tool 20 and the anatomical volume AV are also shown for illustrative purposes. In other instances, the tool 20 and the anatomical volume AV may include a different shape and/or size.

The feed rate generator 82 may identify the quantity of pixels PX within the intersection I using a variety of methods. In one example, only pixels PX that are entirely located within the intersection I are considered, while disregarding partial intersections. In some instances, the feed rate generator 82 may consider pixels PX that are at least partially located within the cross-sectional intersection. For example, the feed rate generator 82 may determine that a pixel PX is located within the cross-sectional intersection I if greater than a predetermined percentage of a pixel PX, e.g., 50%, is located within the cross-sectional intersection I. For example, as shown in FIG. 8, the feed rate generator 82 may identify that pixels PX1, PX2, PX5, PX6 are partially located within the cross-sectional intersection I. In another example, a ratio of intersection can be applied to each pixel PX depending on how much of the pixel is within and outside of the intersection.

In some instances, the feed rate generator 82 may filter any intersecting pixels PX based on a density value DV of a pixel PX. For example, referring to FIG. 7, each pixel PX of slices A, B, C, D includes a corresponding density value DV of the anatomical data 65. The feed rate generator 82 may evaluate whether the density value DV of the intersecting pixel PX exceeds or falls below a predetermined threshold. If so, the feed rate generator 82 can include or disregard the intersecting pixel PX based on such values.

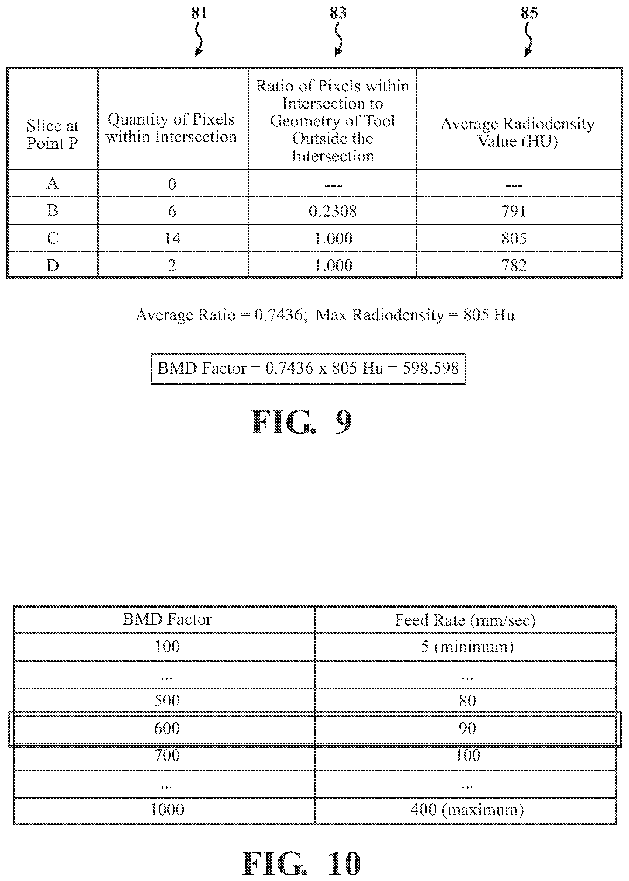

The feed rate generator 82 may store the quantity of pixels PX within the intersection I for each slice A, B, C, D. FIG. 9 provides a visual representation of the stored quantities. As shown, the quantity of pixels PX within the intersection I may be stored in column 81 for each slice A, B, C, D.

Any aspect of the techniques described herein that involves using pixels PX can be implemented in other ways that do not involve pixels PX. For instance, voxels of the anatomical volume AV may be determined from a 3-D model of the anatomy and compared to a 3-D model of the tool geometry at the points P. The voxels can include density values DV. Any other type of 2-D or 3-D unit or measurement can be utilized when comparing the geometries described herein.

During step M7, the feed rate generator 82 identifies the density values DV of the anatomical data 65 within the intersection I. The density values DV illustrate a density of the anatomical volume AV at the intersection I. For example, the density values DV allow the feed rate generator 82 to determine how the tool 20 is contacting different types of tissue, such as compact bone tissue, cancellous bone tissue, subchondral bone tissue, fat, and/or cartilage. Additionally, a null or zero value may indicate a region of air. In this way, the cut plan data 79 accounts for a type of tissue contacted at a point P. More particularly, the cut plan data 79 includes a planned feed rate factor FR that accounts for the type of tissue contacted at a point P. The density values DV can be radiodensity values DV measured in Hounsfield Units. However, in other instances, the density values DV may include units other than Hounsfield units (HU). For instance, density values DV can be any measure of radiopacity, radiolucency, and/or transradiancy.

Continuing with the example of FIG. 7, the feed rate generator 82 may identify the density values DV of the anatomical data 65 within the intersection I for each slice A, B, C, D. The feed rate generator 82 can disregard any density values for pixels/voxels deemed to be outside of the intersection I. In FIG. 7, each pixel PX of anatomical slices A, B, C, D includes a corresponding radiodensity value DV. Specific, but non-limiting, examples of radiodensity values DV for slice B at point P1 are shown in FIG. 8, for example. As shown, the intersection I includes pixels PX1-PX6, and the feed rate generator 82 identifies radiodensity values DV 795 HU, 780 HU, 785 HU, 790 HU, 795 HU, and 801 HU, respectively. The feed rate generator 82 may similarly determine the density values DV of the anatomical data 65 within the intersection I for the other slices C, D.

Once the feed rate generator 82 identifies the density values DV within the intersection I for each slice A, B, C, D, the feed rate generator 82 collects the density values DV. The feed rate generator 82 may collect the density values DV by computing or determining one or more of: an average, a median, range, mode, a maximum, or a minimum density value DV of the density values DV for each slice A, B, C, D. For example, in the instance of FIG. 8, pixels PX1-PX6 of slice B have an average radiodensity value DV of 791 Hounsfield units (HU). The feed rate generator 82 may then store the average radiodensity value DV of pixels PX1-PX6 in memory, as shown in column 83 of FIG. 9. The feed rate generator 82 may similarly collect and store the density values DV within the intersection I for slices C, D.

During step M8, the feed rate generator 82 can compute a tool contact factor that relates to an extent to which the tool contacts the anatomical volume AV at the identified point. For example, the tool contact factor can expose whether the tool 20, at the given point, brushes against or grazes the anatomical volume AV or whether the tool 20 burrs into the anatomical volume AV or is performing any air cutting. In this way, the cut plan data 79 accounts for the extent of contact between the tool 20 and the anatomical volume AV at the point P. The cut plan data 79 includes a planned feed rate factor FR that accounts for the extent of the tool contact at a point P.

In one example, the tool contact factor is an intersection ratio related to the geometry of the tool 20 within the intersection I relative to the geometry of the tool 20 outside of the intersection I at a point P. As will be described herein, the intersection ratio may be determined based on the quantity of pixels within the intersection I for each slice A, B, C, D. However, in other instances, the intersection ratio may be determined using any other suitable method.

The feed rate generator 82 may determine the intersection ratio by determining individual intersection ratios for each slice A, B, C, D. The feed rate generator 82 computes the individual intersection ratios by computing a ratio between a first quantity of the geometry of the tool 20 that is located within the intersection I and a second quantity of the geometry of the tool that is located beyond the intersection for each slice A, B, C, D. For example, the feed rate generator 82 may compute the intersection ratios based on a quantity of pixels PX of the tool 20 located within the intersection I and a quantity of pixels PX of the tool 20 located beyond the intersection I. In other instances, the feed rate generator 82 may compute the intersection ratios based on a quantity of pixels PX of the tool 20 located within the intersection I and a total number of pixels PX of the tool 20.

Referring to the example of Slice B in FIG. 8, the feed rate generator 82 may determine that the energy applicator 24 at this slice occupies or intersects twenty-six total pixels, with six pixels located within the intersection I and twenty pixels located beyond the intersection. As such, the feed rate generator 82 may calculate the intersection ratio for slice B to be (6/26) or 0.2308. Other ways of computing similar ratios are contemplated and need not necessarily be a ratio of intersecting pixels/total pixels of the tool 20. The feed rate generator 82 may then store the intersection ratio for slice B in column 85 of FIG. 9. The feed rate generator 82 may similarly determine and store the intersection ratio for slices C, D. In another example, the intersection ratio is based on voxel intersection in the same manner described with respect to pixels.

The tool contact factor can alternatively or additionally be based on other computations. For example, the feed rate generator 82 can evaluate the surface, circumference, perimeter, outline, or 2-D or 3-D boundary of the tool geometry relative to the anatomical volume. For example, the feed rate generator 82 can determine in FIG. 8 what amount of the circumference of the tool geometry is passing through or touching the anatomical volume AV as compared with air. This amount can be a length (e.g., arc length), degree measurement, or ratio of the geometrical boundary. Alternatively, an area or volume of the tool geometry can be evaluated relative to the anatomical volume AV, with or without regard to any pixels/voxels. The feed rate generator 82 can supplement a geometrical approach to evaluating the tool contact by evaluating or comparing a hardness of the tool relative to density values DV of the anatomical volume AV. This tool hardness evaluation can be used to determine individual contact forces that may be applied to each pixel/voxel.

During step M10, the feed rate generator 82 sets a planned feed rate factor FR for the tool 20 at a path segment PS corresponding to the point P based on the determined density values DV and the computed tool contact factor. In some instances, it is contemplated that the feed rate generator 82 can set the planned feed rate factor FR for the tool 20 based on the computed tool contact factor, without regard to the determined density values DV. During step M10, the feed rate generator 82 may optionally calculate a bone mineral density (BMD) factor based on the determined density values DV and the computed tool contact factor. The feed rate generator 82 then sets the planned feed rate factor for the tool based on the calculated BMD factor.

The feed rate generator 82 may calculate the BMD factor by multiplying the radiodensity values DV collected for each intersection slice during step M7 by the tool contact factor calculated for each intersection slice during step M8. To calculate the BMD factor in this way, the feed rate generator 82 converts the radiodensity values DV that were previously collected for each intersection slice during step M7. Referring to FIG. 9, the feed rate generator 82 converts the radiodensity values DV by calculating a maximum of the collected radiodensity values DV (805 HU). In other instances, the feed rate generator 82 may convert the radiodensity values DV using an average, median, and/or minimum radiodensity value DV. The feed rate generator 82 also converts the tool contact factors that were previously calculated for each slice during step M8. Referring to the example of FIG. 9, the feed rate generator 82 can convert the tool contact factors by calculating an average of the intersection ratios (0.7436). In other instances, the feed rate generator 82 may convert the radiodensity values DV using a median, maximum, and/or minimum radiodensity value DV. As follows, the feed rate generator 82 calculates the BMD factor to be 598.598.

In some instances, the BMD factor is utilized as a standardization factor to account for variable density values DV, whether such density values are normalized or raw density values. For example, based on the density values DV, the BMD factor may be chosen from a standardized scale between 1 and 100. The standardized scale may take into account different normalization factors depending on, e.g., the imaging modality, the scanner used to image the anatomical volume, parameters of a density phantom, or measured forces on the tool, as described above.

The feed rate generator 82 may access a look-up table to determine the planned feed rate factor FR based on the calculated BMD. Specifically, the feed rate generator 82 may access a look-up table defining associations between predefined BMD factors and predefined feed rate factors FR. An example look-up table is shown in FIG. 10. The feed rate generator 82 identifies, in the look-up table, the predefined BMD factor that is closest to the calculated BMD factor and sets the planned feed rate factor FR based on the predefined feed rate factor associated with the closest identified predefined BMD factor. In the example of FIG. 9, the feed rate generator 82 calculates the BMD factor to be 598.598. As such, the feed rate generator 82 may determine that the predefined BMD factor of 600 is closest to 598.598. The feed rate generator 82 may then set the planning feed rate factor FR for the path segment PS1 to be 90 mm/sec.

The look-up table shown in FIG. 10 serves as an example look-up table. In other instances, the planned feed rate factors FR and the BMD factors may vary based on a preference for optimizing cutting performance. For example, in instances where the control system 60 is more or less sensitive to variations in the density values DV of the anatomical volume AV, a difference between successive BMD factors in the look-up table may be reduced or increased. As another example, in instances where the control system 60 reduces or increases variations in the planned feed rate factor FR, a difference between successive planned feed rate factors FR may be reduced or increased. Furthermore, while the look-up table in FIG. 10 features evenly spaced BMD factors (from 100 to 1000), in some instances, the look-up table may include additional BMD factors and corresponding planned feed rate factors FR around a predetermined range (e.g., 505, 510, 515, 520, etc.) to increase a resolution of determining a planned feed rate factor FR around the predetermined range of BMD factors.

In some instances, the feed rate generator 82 may set the planning feed rate factor FR for a path segment PS to be a maximum feed rate factor in response to determining that the calculated BMD factor is below a minimum threshold. For example, referring to the look-up table of FIG. 10, the feed rate generator 82 may set the planning feed rate factor FR to be 5 mm/sec (a minimum feed rate of the tool 20) if the BMD factor is less than 100. In some instances, setting the planned feed rate factor to be a minimum feed rate factor in response to determining that the calculated BMD factor is above a maximum threshold. For example, referring to the look-up table of FIG. 10, the feed rate generator 82 may set the planning feed rate factor FR to be 400 mm/sec (a maximum feed rate of the tool 20) if the BMD factor is greater than 1000. In practice, the feed rate FR of the tool 20 is typically between 5 and 400 mm/sec. However, the feed rates FR can be greater or less than this range depending on the situation.

In some instances, the feed rate generator 82 may obtain a default planned feed rate factor FR for each path segment PS. For example, the feed rate generator 82 may, after obtaining the path data 73 during step M1, set a default planned feed rate factor FR for each path segment PS of the tool path TP. During step M8, the feed rate generator 82 may modify the default feed rate FR for a path segment PS based on the calculated BMD factor. For example, referring to FIG. 10, the feed rate generator 82 may obtain a default planned feed rate factor FR of 90 mm/sec for each path segment PS along a tool path TP. The feed rate generator 82 may then modify the default planned feed rate FR from 90 mm/sec to a different planned feed rate (e.g., 80 mm/sec, 100 mm/sec) based on the calculated BMD factor. In this way, the feed rate generator 82 may retain the default planned feed rate FR for some path segments PS of the tool path TP. The look-up table of FIG. 10 may vary based on a desirability of the user to modify the planned feed rate FR from the default planned feed rate FR. For example, in instances where the user prefers to leave the planned feed rate FR as the default planned feed rate FR, a difference between successive BMD factors may be increased. In such instances, additional BMD factors may be set to correspond to the default planned feed rate FR. In instances where the user prefers to modify the planned feed rate FR from the default planned feed rate FR, a difference between successive BMD factors may be reduced. In such instances, a number of BMD factors corresponding to default planned feed rate FR may be reduced.

### d. Disregarding Previously Analyzed Anatomical Regions

During step M9, the feed rate generator 82 is optionally configured to disregard portions (pixels/voxels/areas/volume) of the anatomical volume VM that were previously evaluated during the process shown in FIG. 4. The purpose of this step is to consider/disregard these previously analyzed regions on the presumption that these previous regions would have been removed by the tool 20 along the tool path TP. Disregarding the density values of previously removed parts of the anatomical volume AV helps generate more accurate feed rate FR determinations for the current point. This approach is useful, for example, when a distance between successive points P along the tool path TP is smaller than a size of the tool 20 or when the tool path TP passes through the same anatomical region twice. In such instances, the feed rate generator 82 need not factor in the density values DV corresponding to the previously removed parts of the anatomical volume AV.

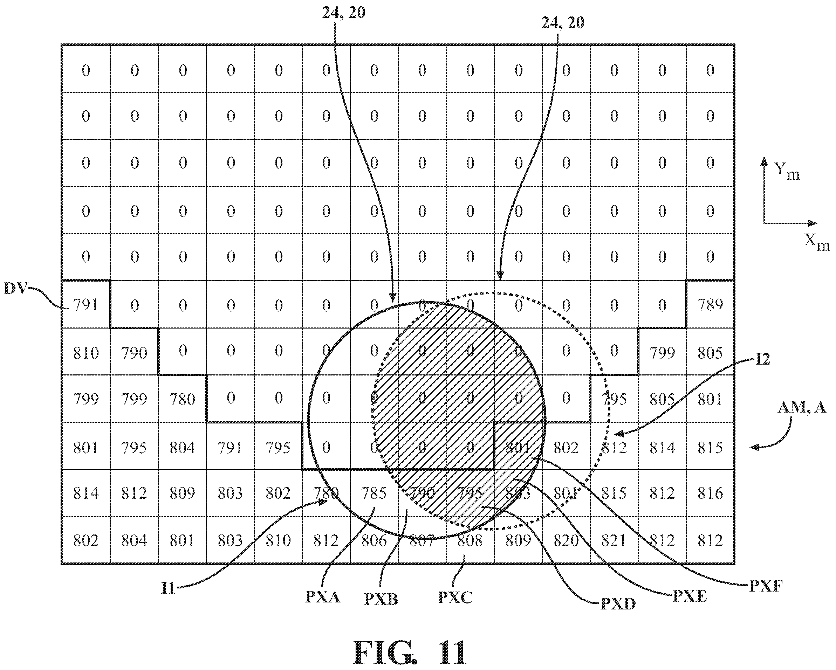

FIG. 11 illustrates an example instance of step M9. FIG. 11 includes a slice of the tool geometry and an imaged slice of the anatomical volume AV when the tool 20 is at a first point P1 and at a successive second point P2 along the tool path TP. The tool 20 at the first point P1 is indicated using a solid line and the tool 20 at the second point P2 is indicated using a dotted line. The tool 20 is shown intersecting the anatomical model AM at the first point P1 at intersection I1 and shown intersecting the anatomical model AM at the second point P2 at intersection I2.

As shown, the respective intersections I1, I2 of the tool 20 and the anatomical volume AV at points P1 and P2 include common pixels PX. These common pixels PX illustrate that, at point P2, the tool 20 is expected to intersect a part of the anatomical volume AV that was previously removed by the tool 20 when the tool 20 was at point P1. As such, the feed rate generator 82 is configured to disregard the common pixels PX when determining the cut plan data 79 for point P2.

In the instance of FIG. 11, the feed rate generator 82 first generates and stores the pixels PX corresponding to intersection I1 for point P1. These pixels PX indicate locations of the intersection I1 and are referred to herein as "interaction coordinates". The feed rate generator 82 can also store density values DV corresponding to the interaction coordinates. These determined density values DV of the intersection I1 will be referred to herein as "previously determined density values DV". These interaction coordinates need not be specifically limited to the specific coordinates of pixels/voxels. Instead, any technique described above in relation to the tool contact factor can be similarly utilized to determine previous interaction of the tool 20 with the anatomical volume AV. Also, the coordinates can be identified relative to a coordinate system (with or without regard to pixel/voxel location), using the tool path TP coordinate system, anatomical coordinate system, or the merged coordinate system.

Continuing with the example of FIG. 11, the feed rate generator 82 has proceeded through steps M4-M8 for the second point P2. During step M9, the feed rate generator 82 disregards the previously determined interaction coordinates. To do this, the feed rate generator 82 first compares interaction coordinates (e.g., pixels PX) for the second point P2 to the interaction coordinates for the first point P1. The feed rate generator 82 may then disregard any density values DV of the second point P2 that have interaction coordinates identical to the interaction coordinates of the first point P1. In FIG. 11, these common interaction coordinates of intersections I1 and I2 include pixels PXA-PXF. As such, the feed rate generator 82 disregards the density values DV associated with pixels PXA-PXF when determining the cut plan data 79 for the second point P2. Disregarding such values can mean that the feed rate generator 82 nullifies or zeroes out these the density values at such coordinates. Furthermore, although the points P1, P2 evaluated in the context of this process were successive points, P2 could be any point subsequent in time to P1, and not necessarily immediately successive to P1.

In some instances, the feed rate generator 82 may be configured to disregard a previously determined density value DV depending on the amount of prior pixel/voxel interaction with the tool geometry. For example, the feed rate generator 82 may be configured to disregard a previously determined density value DV if more than X% of the pixel PX corresponding to the previously determined density value DV was previously interacted with. For example, referring to FIG. 11, pixel PXC may not be disregarded because less than 50% of pixel PXC was interacted with by the tool 20 at the first point P1. However, pixels PXA, PXB, PXD, PXE, PXF may be disregarded because greater than 50% of pixels PXA, PXB, PXD, PXE, PXF were interacted with by the tool 20 at the first point P1. In another instance, the feed rate generator 82 may be configured to disregard a previously determined density value DV only if 100% of the pixel PX associated with the previously determined density value DV was interacted with. For example, referring to FIG. 11, pixels PXA, PXC, PXE, PXF may not be disregarded because less than 100% of pixels PXA, PXC, PXE, PXF were interacted with by the tool geometry at the first point P1. However, pixels PXB, PXD may be disregarded because 100% of pixels PXB, PXD were interacted with by the tool geometry at the first point P1. In other examples, the tool contact factor can be considered for purposes of determining whether or not to disregard prior interaction coordinates.

In other instances, instead of disregarding density values DV at previous interaction coordinates, the feed rate generator 82 may be configured to use a scaled version or residue of the previously determined density value DV. The scaled previously determined density value DV may be based on a measure of the previous interaction. For example, referring to FIG. 11, the tool geometry interacted with approximately 25% of pixel PXC at point P1. As such, instead of disregarding pixel PXC from the determination of the cut plan data 79 for point P2, the feed rate generator 82 may scale the density value DV corresponding to pixel PXC based on the interaction at point P1. For example, the feed rate generator 82 may determine the cut plan data 79 for point P2 using 75% of the density value DV corresponding to pixel PXC (606 HU).

### e. Outputting the Cut Plan Data

During step M11, the feed rate generator 82 outputs the cut plan data 79 for the tool path TP. Referring to FIG. 3, the feed rate generator 82 may output the cut plan data 79 to the behavior controller 74. The behavior controller 74 may then control the motion controller 76 based on the cut plan data 79. In this way, the control system 60 controls the manipulator 14 to move the tool 20 along the tool path TP at the planned feed rates FR based on the cut plan data 79. Additionally, referring to FIG. 3, the cut plan data 79 may be output to the clinical application 80. As such, a user operating the clinical application 80 may view the cut plan data 79 prior to performing or during a surgical procedure. Furthermore, a user operating the clinical application 80 may adjust the cut plan data 79 prior to performing or during a surgical procedure.

As shown in FIG. 6, the cut plan data 79 includes the tool path TP. However, the cut plan data 79 also includes planned feed rate factors FR1...FRN for path segments PS1...PSN along the tool path TP. In this way, the cut plan data 79 may be an enhanced or augmented version of the originally obtained tool path TP. Additionally, in some instances, once the feed rate generator 82 generates the cut plan data 79, the cut plan data 79 may replace the original tool path TP.

Furthermore, in some instances, the feed rate generator 82 may evaluate the cut plan data 79 to determine that it is prudent to alter the original tool path TP. Such alteration can be based on evaluating any consideration, factor or step described herein, such as the planned feed rate factors FR1...FRN, the tool contact factors, disregarded interaction coordinates, etc. For example, the feed rate generator 82 can alter the tool path TP to optimize cutting performance, cut time, and the like. For instance, if the planned feed rate FR at one point is excessively slow or fast, the feed rate generator 82 can alter the tool path TP by changing the location of the point or removing the point altogether. In another example, if the tool contact factor at one point is excessively high or low, the feed rate generator 82 can alter the tool path TP by changing the location of the point. In another example, if at one point P, the feed rate generator 82 detects an excessive amount of identified previous interaction from a previous point, the feed rate generator 82 can alter the tool path TP by changing the location of the point P or the previous point so as to reduce cutting time. In an instance where the path data 73 includes a set of potential tool paths TP, the feed rate generator 82 may determine which of the potential tool paths TP optimizes cutting performance, cut time, and the like. The feed rate generator 82 may then select the determined tool path TP for the cut plan data 79 while disregarding the other tool paths TP.

The altered tool path TP can be automatically determined and implemented by the feed rate generator 82. Alternatively, the proposed alteration can be presented to the user for confirmation.

In some instances, cutting of a resection volume during a surgical procedure may be repeated until the control system 60 determines that the resection volume is fully removed. In some instances, the tool 20 may encounter an error such that the tool 20 may not follow the original tool path TP, resulting in the resection volume not being fully removed. In other instances, an altered tool path TP, when followed by the tool 20, may not fully remove the resection volume. In such instances, cutting of the resection volume may be repeated (e.g., after the error and/or after the tool 20 moves according to the altered tool path TP) by restarting the surgical procedure and moving the tool 20 according to the original tool path TP or according to an altered tool path TP. The surgical procedure may be restarted and repeat as many times as necessary until the resection volume is fully removed. In some instances, the user may be notified that the resection volume is not fully removed and may be presented with an option of restarting and repeating the surgical procedure according to the original tool path TP, or according to an altered tool path TP.

### f. Additional Implementations

In some implementations, the cut plan data 79 may be determined based additionally or alternatively on a tissue parameter. In such implementations, the anatomical data 65 may include a tissue parameter, which may include any tissue parameter data. For example, the tissue parameter may include density values DV of the anatomical volume AV, and/or may include other tissue parameter data. For example, a type of tissue of the anatomical volume AV may be determined from the tissue parameter data. In such an example, the control system 60 may determine that a tissue of the anatomical volume AV is cancellous, cortical, cortical, cartilage, or soft tissue based on the tissue parameter data. Tissue parameter data can also include an electrical parameter of the tissue, such as permittivity or conductivity. Tissue parameter data can also include blood flow parameter, volume, voxel size, area, pixel size, thickness, cortical thickness, weight, fat fraction, total fat, heat parameters, thermal conductivity, thermal expansion, tensile strength, acoustic parameters, elasticity, bone surface density, bone surface/volume ratio, and the like.

In some implementations, the cut plan data 79 may be determined based on a predefined pose of the shaft 33. For example, a predefined pose of the shaft 33 may affect the simulated interaction I between the geometry of the tool 20 and the anatomical volume AV at a point P. Specifically, a predefined pose of the shaft 33 may affect how the tool 20 contacts the anatomical volume AV at the intersection I depending on the geometry of the tool 20. For example, in an instance where a cutting surface of the energy applicator 24 includes a triangular or tapered cross-section or a flat cutting tip, the predefined pose of the shaft 33 affects the intersection I of the tool 20 and the anatomical volume AV. As such, to more accurately identify the intersection I during step M6, the control system 60 may be configured to obtain the path data 73 by obtaining a planned pose of the TCP during step M1.

In some implementations, the cut plan data 79 may include an output pose of the shaft 33 associated with at least one point P of the tool path TP. In such implementations, the control system 60 determines the output pose for the shaft 33 as the energy applicator 24 travels along one or more individual path segments PS of the tool path TP to optimize cutting performance. The output pose of the shaft 33 may be a state (e.g., a position and/or orientation) of the shaft 33 with respect to the manipulator coordinate system MNPL. By outputting the output pose of the shaft 33, the control system 60 provides the manipulator controller 26 with instructions for 14 for moving the tool 20.

The control system 60 may determine the output pose of the shaft 33 based on the anatomical data 65 (e.g., density values DV and/or a tissue parameter), the path data 73, and the tool data 69. For example, the control system 60 may determine the output pose based on the geometry of the tool 20. For instance, the cutting surface of the energy applicator 24 may include a tapered cross-sectional area. In such an instance, the control system 60 may determine the output pose to position and orient the tool 20 to optimize cutting with the tapered cross-sectional area. As another example, the control system 60 may determine the output pose based on a preferred use of the tool 20. For instance, the cutting surface of the energy applicator 24 may be positioned at a side of the energy applicator 24 to promote cutting using sides of the energy applicator 24, instead of a tip of the energy applicator 24. In such an instance, the control system 60 may determine the output pose to position and orient the tool 20 such that the side of the energy applicator 24 performs a majority of the cutting. As yet another example, the control system 60 may determine the output pose based on a preferred cutting depth of the tool 20. For instance, the tool 20 may be preferably used for shallower cuts. In such an instance, the control system 60 may determine the output pose to position and orient the tool 20 to perform a shallower cut.

The control system 60 may set the output pose of the shaft 33 during step M10 of the above-described method. Furthermore, the control system 60 may output the cut plan data 79, including the output pose, during step M11. Additionally, the control system 60 may execute the above-described steps M1-M9 to determine the output pose. The control system 60 may output the output pose of the shaft 33 in addition to, or as an alternative to, outputting the planned feed rate FR of the tool 20.

In some implementations, the feed rate generator 82 may determine the planned feed rate FR and/or adjust the cut plan data 79 to achieve a predetermined motor current on a motor of the tool 20. For example, the feed rate generator 82 may determine the planned feed rate FR and/or adjust the cut plan data 79 to maintain a constant motor current on a motor of the tool 20. As another example, the feed rate generator 82 may determine the planned feed rate FR and/or adjust the cut plan data 79 to ensure that a motor current on a motor of the tool 20 is within a predetermined range of motor current values.

In some implementations, the control system 60 may monitor a performance of the tool 20 during a surgical procedure. Specifically, the control system 60 may monitor a performance of the tool 20 while the tool 20 cuts a resection volume of the anatomical volume AV according to the cut plan data 79. The control system 60 may monitor a contact force of the tool 20 applied to the anatomical volume AV, a rotational speed of the tool 20, an actual feed rate FR of the tool 20, a temperature of the tool 20, a current of a motor of the tool 20, and/or any other suitable measure of performance of the tool 20. The control system 60 may then modify the cut plan data 79 based on monitoring the performance of the tool 20. For example, the control system 60 may modify the density values DV of the anatomical volume AV, the planned feed rate FR, the tool path TP, the BMD factors, a geometry of the tool 20, and/or any other parameter used in the determination of the cut plan data 79.

### III. Density Value Parameterized Virtual Object

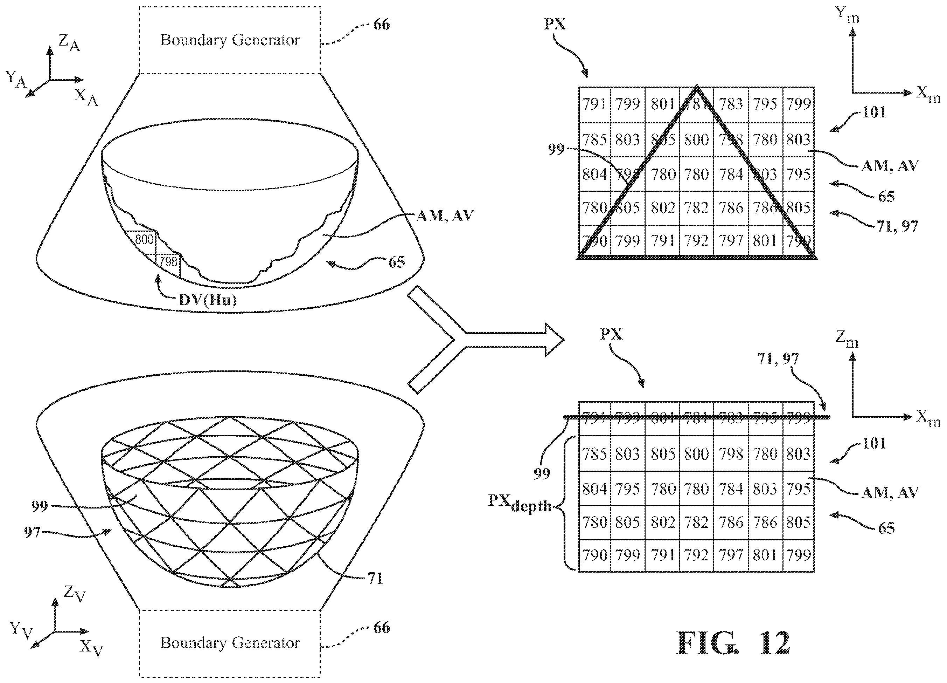

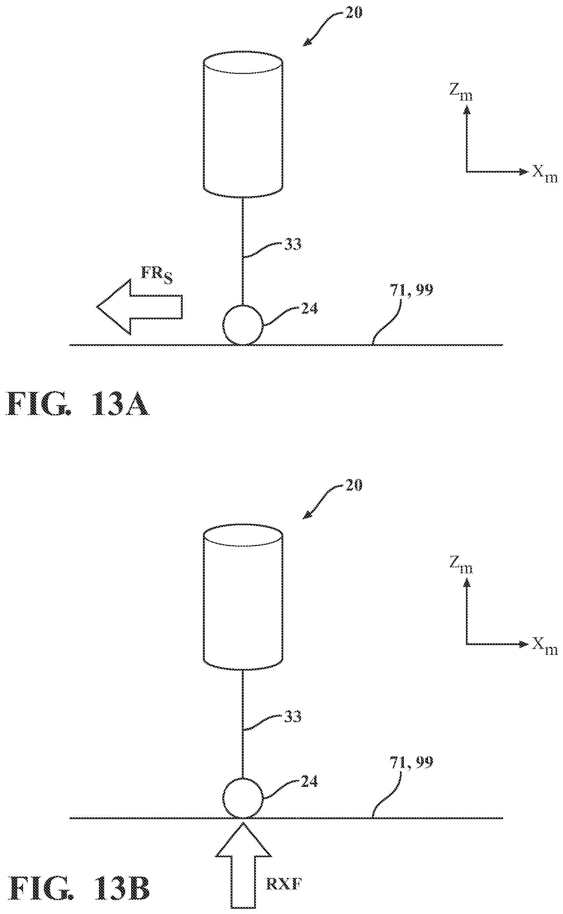

With reference to FIGS. 12 and 13, described herein are techniques for the control system 60 to create or modify a virtual object 71 such that the virtual object 71 is characterized by, coded with, or otherwise associated with properties, parameter(s) or factors derived from the density values DV. This virtual object 71 can be used by the control system 60 to modify an operation of the manipulator 14 or the tool 20 based on the tool interacting with the virtual object 71.

As described above, the state of the tool 20, TCP, or energy applicator 24 can be measured relative to the virtual object 71 for purposes of determining forces to be applied to a virtual model of the tool 20 in a virtual simulation. In the virtual simulation, model of the tool 20 can be represented as the virtual rigid body model or virtual volume. Forces are computed with respect to the virtual model in response to the virtual model interacting with one or more polygonal elements 99 of the virtual object 71. These forces applied to the virtual model result in an output which the control system 60 can use to modify operation of the manipulator 14 or the tool 20. In response, the control system 60 may compute a reactive force to modify an operation of the manipulator 14 by preventing the tool 20 from penetrating the virtual object 71. In response to the interaction, the control system 60 can also modify a feed rate of the tool 20. The techniques evaluating tool interaction with a virtual object 71 can be like that described in U.S. Patent Publication No. 2018/0353253, filed on June 6, 2018, and entitled "Robotic Surgical System And Method For Producing Reactive Forces To Implement Virtual Boundaries".

Tool interaction with the virtual object 71, and the techniques described herein, can occur, or be implemented in any mode of operation for the manipulator 14, including autonomous, manual, or guided-manual modes. The autonomous and manual modes have been described above. In the guided manual mode, the control system 60 obtains the tool path TP for the tool 20 and defines virtual constraints on movement of the tool 20 along the tool path TP with respect to two degrees of freedom each being normal to the tool path TP. In this way, the virtual constraints are defined to constrain movement of the tool 20 to be along the tool path TP. Additionally, in the guided manual mode, the control system 60 receives an input from the force/torque sensor S in response to user forces and torques manually applied to the tool 20 by a user. The control system 60 then simulates dynamics of the tool 20 in the virtual simulation based on the virtual constraints and the input from the force/torque sensor S and commands the manipulator 14 to advance the tool 20 along the tool path TP based on the virtual simulation. For example, the control system 60 may compute and simulate a reactive force to the virtual object 71 in the virtual simulation and commands the manipulator **14 to advance** the tool 20 along the tool path TP based on the virtual simulation. The guided manual mode is further described in U.S. Patent Publication No. 2020/0281676.

According to this technique, the control system 60 obtains the anatomical data 65 and the virtual object data 97 associated with the virtual object 71. The control system 60 obtains anatomical data 65, which includes a geometry and density values DV of the anatomical volume AV. The anatomical data 65 is illustrated in FIG. 12 as the anatomical model AM of the anatomical volume AV, including density values DV. The anatomical data 65 can be mapped on an anatomical data coordinate system X_{A}, Y_{A}, Z_{A}. The control system 60 also obtains virtual object data, which includes a geometry of the virtual object 71 having a mesh of polygonal elements. The virtual object data 97 is illustrated in FIG. 12 includes the virtual object 71, which is mapped on a virtual object coordinate system X_{V}, Y_{V}, Z_{V}. As shown, the virtual object 71 includes a mesh of polygonal elements 99. In FIG. 12, the polygonal elements 99 are triangular. However, in other instances, each individual polygonal element 99 may be any suitable polygon.

Referring to FIG. 12, the control system 60 merges the anatomical data 65 and the virtual object data to create a merged model 101, which can be mapped on a merged coordinate system X_{M}, Y_{M}, Z_{M}, or any other coordinate system described, such as the anatomical data coordinate system X_{A}, Y_{A}, Z_{A}, the virtual object coordinate system X_{V}, Y_{V}, Z_{V}, or a new, different, or arbitrary coordinate system. This merger can, but need not be, provided or performed in visual form. For instance, the merger can be purely computational and unseen to a user. Alternatively, aspects of these steps can be visualized for the user, using e.g., the clinical application 80 so that the user can experience, or interact with the virtual object 71 and the anatomical data 65.

FIG. 12 provides two views of the merged model 101: a first view where the merged model 101 is shown in the X_{M}, Y_{M} coordinate system, and a second view where the merged model 101 is shown in the X_{M}, Z_{M} coordinate system. In both views, a polygonal element 99 of the virtual object 71 and the anatomical model AM are superimposed.

For illustrative purposes, the polygonal element 99 shown may be exaggerated relative to the size of the density values DV regions, and hence, not to scale. Deepening on the size of the polygonal elements 99 and density value DV regions, any number of polygonal elements 99 may fit inside one density value DV region, and any number of density value DV regions may fit inside one polygonal element 99.

Once merged, the virtual object 71 can intersect the anatomical volume AV and/or can be spaced apart from the anatomical volume AV. Any number of virtual objects 71 may be utilized in this technique and these virtual objects 71 can have any suitable shape (e.g., planar, or complex shapes) or purpose (e.g., keep-out zone, keep-in zone, boundary, etc.). In one example, the virtual object 71 can be like boundaries described in US20160338782A1, filed on May 18, 2016, entitled, "System And Method For Manipulating An Anatomy". The virtual object 71 can have any number of polygonal elements 99. The virtual object 71 may generated by the boundary generator 66, as described above.

Once the control system 60 merges the anatomical data 65 and the virtual object 71, the control system 60 determines a parameter to associate with one or more polygonal elements 99 of the virtual object 71. This parameter can define an effect, an influence, and/or a reaction that a polygonal element 99 has on the virtual model of the tool as applied in the virtual simulation. The parameter can define a first parameter of the polygonal element 99 or the parameter can over-write or replace an existing parameter. In one example, the parameter is a tuning parameter related to stiffness of the polygonal element 99. This tuning parameter can define spring or damper parameters related to the polygonal element 99. In another example, the parameter can influence the feed rate of the tool 20. For example, the feed rate generator 82 may be coupled to the behavior controller 74 and can be made aware of any interaction between the model of the tool 20 with any polygonal element 99 parameterized as described herein. From there, the feed rate generator 82 can define or adjust the feed rate of the tool 20 based on such interaction.

Although pixels PX are used as an example below, any aspect of the techniques described herein that involves using pixels PX can be implemented in other ways that do not involve pixels PX. For instance, voxels VX of the anatomical volume AV may be used as well. The voxels VX can include density values DV. Any other type of 2-D or 3-D unit or measurement can be utilized when comparing the geometries described herein.

Referring to the first view of the merged model 101 of FIG. 12, the control system 60 in one example may determine the parameter of illustrated polygonal element 99 by accounting for the density values DV of the pixels PX that are encapsulated by a perimeter of the polygonal element 99 in the X_{M}, Y_{M} coordinate system. The control system 60 can also determine the parameter for the polygonal element 99 by accounting for the density values DV of the pixels PX at least partially encapsulated by the polygonal element 99 in the X_{M}, Y_{M} coordinate system. In other instances, the control system 60 may determine the parameter for the polygonal element 99 by accounting for the density values DV of the pixels PX that are completely encapsulated by the polygonal element 99. The control system 60 may also disregard the density values DV of the pixels PX if less than X% of the pixel PX is encapsulated by the polygonal element 99.

Referring to the second view of the merged model 101 of FIG. 12, the control system 60 may additionally, or alternatively, determine the parameter of polygonal element 99 by accounting for the density values DV of the pixels PX that are intersected by a plane of the polygonal element 99 in the X_{M}, Z_{M} coordinate system. The control system 60 may determine the parameter for the polygonal element 99 by accounting for the density values DV of the pixels PX at least partially intersected by the polygonal element 99. In other instances, the control system 60 may determine the parameter for the polygonal element 99 by accounting for the density values DV of the pixels PX within a predetermined or measured depth or distance from the polygonal element 99, shown as PX_{depth} shown in FIG. 12. The depth PX_{depth} may be any suitable depth and be in any suitable units. For example, the depth PX_{depth} may be predetermined, e.g., 1mm below the virtual object 71 or may be 3 pixels PX below the virtual object 71.

In some instances, the control system 60 may determine the parameter for the polygonal element 99 using density values DV of pixels PX below the virtual object 71 depending on how the tool 20 or the virtual model of the tool 20 penetrates the virtual object 71. In such instances, the control system 60 may account for the density values DV of the pixels PX below the virtual object 71 that are interacted with by the tool 20 or a virtual model of the tool 20. For example, the control system 60 may account for any density values DV that intersect the virtual model of the tool 20.

Once the control system 60 identifies the density values DV that should be accounted for, the control system 60 may determine the parameter for the polygonal element(s) 99 in many ways, including, but not limited to, by computing or determining one or more of: an average, a median, range, mode, a maximum, or a minimum density value DV of the density values DV that are proximate to, interacting with, related to, or intersecting the polygonal elements 99. Although the parameter value is based on the density values DV, the parameter value need not correspond to a density value resulting from the computation related to the accounted for density values DV. Instead, the parameter value can be a standardized or normalized value.

Once the control system 60 determines the parameter for the polygonal elements 99 of the virtual object 71, the control system 60 may modify operation of the tool 20 in response to the tool 20 interacting with the polygonal elements 99.

In the example of FIG. 13A, the energy applicator 24 exhibits a scenario where it slides along the virtual object 71 at a first feed rate. The first feed rate can be predetermined or based on a user applied force, where the manipulator is controlled in a manual or guided-manual mode in response to user applied force. In these modes, the user has freedom to move the tool 20 in a way which may cause the tool 20 to slide along the virtual object 71. In turn, this may result in the virtual model of the tool 20 skimming or partially penetrating the polygonal elements 99 in the virtual object 71. In such instances, the control system 60 may compute a second feed rate according to the parameter of polygonal elements 99 that the tool 20 interacts with. The control system 60 may then command the robotic manipulator to move the tool 20 at the second feed rate. For example, a user may move the tool 20 along the virtual object 71 at the illustrated direction with the first feed rate of 2mm/s. The polygonal element 99 with which the tool 20 interacts during this motion may have a parameter of 0.5, which may be a one-half feed rate scaling factor. In response, the control system 60 may control the manipulator 14 according to the second feed rate, which will be one-half of the first feed rate, i.e., 1mm/s. The parameter, hence, is can scale an existing feed rate so that, for example, the density of the anatomy is considered for tool 20 interaction with the virtual object 71.

In the example of FIG. 13B, the energy applicator 24 penetrates the polygonal element 99 of the virtual object 71. In such an instance, the control system 60 modifies operation of the tool 20 based on applying a reactive force RXF derived from the virtual simulation between the model of the tool and the polygonal element(s) 99. The control system 60 computes the reactive force RXF according to the parameter of polygonal element(s) 99 that the tool 20 interacts with and applies the reactive force RXF to the virtual model in the virtual simulation to reduce penetration of the polygonal elements 99 by the virtual model. The control system 60 then commands the manipulator 14 to move the tool 20 in accordance with application of the reactive force RXF to constrain movement of the tool 20 relative to the virtual object 71. This reactive force parameter technique can be utilized separately, or in combination with the feed rate parameter technique.

Several embodiments have been described in the foregoing description. The embodiments discussed herein are not intended to be exhaustive or limited to any particular form. The terminology which has been used is intended to be in the nature of words of description rather than of limitation. Furthermore, the headings used in this document are introduced solely for reference and readability purposes and should not be understood to limit the content of the section solely by the subject matter of the heading.

## Claims

1. A computer-implemented surgical planning method comprising:
obtaining anatomical data (65) comprising a geometry and density values (DV) of an anatomical volume (AV);
obtaining path data (73) comprising a tool path (TP) along which a robotic manipulator will move a tool (20) to interact with the anatomical volume (AV), the tool path (TP) defined by points (P) between which the tool (20) will successively pass;
obtaining tool data (69) including a geometry of the tool (20) that will interact with the tool path (TP);
merging the path data (73) and the anatomical data (65); and
for at least one point (P) of the tool path (TP):
identifying a location of the point (P) relative to the anatomical data (65),
loading, from the tool data (69), the geometry of the tool (20) at the identified location,
at the identified location, identifying an intersection (I) between the geometry of the tool (20) and the anatomical volume (AV), and
determining density values (DV) of the anatomical data (65) within the intersection (I);
**characterized by** the method further comprising for the at least one point (P) of the tool path (TP):
computing a tool contact factor related to an interaction between the geometry of the tool (20) and the anatomical volume (AV),
setting a planned feed rate factor (FR) for the tool (20) based on the determined density values (DV) and the computed tool contact factor, and
associating the planned feed rate factor (FR) with the at least one point (P); and
outputting cut plan data (79) comprising the tool path (TP) including the planned feed rate factor (FR) associated with the at least one point (P) of the tool path (TP).

2. The computer-implemented surgical planning method of claim 1,
wherein obtaining anatomical data (65) further includes obtaining a bone model associated with the anatomical volume (AV); and, optionally,
wherein obtaining path data (73) further includes obtaining the tool path (TP) being predetermined based on one or more of:
a planned resection volume of the bone model of the anatomical volume (AV);
and
geometry of an implant model selected for the bone model.

3. The computer-implemented surgical planning method of claim 1, wherein obtaining anatomical data (65) further includes obtaining imaging data including slices of the anatomical volume (AV).

4. The computer-implemented surgical planning method of claim 3, wherein obtaining imaging data further includes obtaining DICOM data comprising intercept and slope values, slice thickness, and patient position at a time of imaging.

5. The computer-implemented surgical planning method of claim 4, wherein at the identified location, identifying the intersection (I) between the geometry of the tool (20) and the anatomical volume (AV) further comprises:
identifying one or more slices of the imaging data exhibiting the intersection (I) between the geometry of the tool (20) and the anatomical volume (AV).

6. The computer-implemented surgical planning method of claim 5, wherein determining density values (DV) of the anatomical data (65) within the intersection (I) further comprises:
for each identified slice, determining radiodensity values (DV) of the imaging data located within the intersection (I); and
collecting radiodensity values (DV) located within the intersections (I) of each of the one or more identified slices.

7. The computer-implemented surgical planning method of claim 6, wherein setting the planned feed rate factor (FR) for the tool (20) based on the determined density values (DV) further comprises:
calculating a bone mineral density (BMD) factor of the anatomical volume (AV) relative to the identified location based on the collected radiodensity values (DV); and
setting the planned feed rate factor (FR) for the tool (20) based on the calculated BMD factor.

8. The computer-implemented surgical planning method of claim 7, wherein:
obtaining imaging data further comprises obtaining CT slices of the anatomical volume (AV); and
the geometry of the tool (20) comprises a 3-D geometry of the tool (20), and
for the at least one point of the tool path (TP):
loading the 3-D geometry of the tool (20) at the identified location, and
at the identified location, identifying CT slices for which there is a cross-sectional intersection (I) between the 3-D geometry of the tool (20) and the anatomical volume (AV);
for each identified CT slice, determining a Hounsfield unit (HU) for each pixel (PX) within the cross-sectional intersection (I); and
collecting the Hounsfield units (HU) from the pixels (PX) within the cross-sectional intersections (I) of each of the identified CT slices.

9. The computer-implemented surgical planning method of claim 7, wherein:
collecting the radiodensity values (DV) located within the intersection (I) of each of the one or more identified slices further includes identifying one or more of: an average, a median, and a maximum radiodensity value (DV) from the collected radiodensity values (DV); and
calculating the BMD factor further includes converting the one or more of: the average, the median, and the maximum radiodensity value (DV) into the BMD factor.

10. The computer-implemented surgical planning method of claim 9, further comprising, for the at least one point (P) of the tool path (TP), and after identifying slices of the imaging data exhibiting the intersection (I) between the geometry of the tool (20) and the anatomical volume (AV), for each identified slice, computing the tool contact factor by computing an intersection ratio related to the geometry of the tool (20) within the intersection (I) relative to the geometry of the tool (20) outside of the intersection (I).

11. The computer-implemented surgical planning method of claim 10,
wherein converting the one or more of: the average, the median, and the maximum radiodensity value (DV) into the BMD factor further comprises multiplying the one or more of: the average, the median, and the maximum radiodensity value (DV) with the intersection ratio (I); or
wherein, for each identified slice, determining a quantity of pixels (PX) within the geometry of the tool (20) having Hounsfield units (HU) exceeding a predetermined threshold.

12. The computer-implemented surgical planning method of claim 1, further comprising:
calculating a bone mineral density (BMD) factor of the anatomical volume (AV) relative to the identified location based on the determined density values (DV); and
wherein setting the planned feed rate factor (FR) for the tool (20) based on the determined density values (DV) further comprises setting the planned feed rate factor (FR) based on the calculated BMD factor.

13. The computer-implemented surgical planning method of claim 12, further comprising:
accessing a look-up table defining associations between predefined BMD factors and predefined feed rate factors (FR); and
wherein, for the at least one point (P) of the tool path (TP), setting the planned feed rate factor (FR) based on the calculated BMD factor further comprises:
identifying, in the look-up table, the predefined BMD factor that is closest to the calculated BMD factor; and
setting the planned feed rate factor (FR) based on the predefined feed rate factor (FR) associated with the closest identified predefined BMD factor in the look-up table.

14. The computer-implemented surgical planning method of claim 13, wherein, for the at least one point (P) of the tool path (P), setting the planned feed rate factor (FR) based on the calculated BMD factor further comprises:
setting the planned feed rate factor (FR) to be a maximum feed rate factor in response to determining that the calculated BMD factor is below a minimum threshold; and/or
setting the planned feed rate factor (FR) to be a minimum feed rate factor in response to determining that the calculated BMD factor is above a maximum threshold.

15. The computer-implemented surgical planning method of claim 1,
wherein at least one of the following conditions (A to E) is fulfilled:
(A) the method further comprising:
for one point (P) of the tool path (TP), storing interaction coordinates obtained from the intersection (I) between the geometry of the tool (20) and the anatomical volume (AV) at the location of the one point (P), the interaction coordinates indicative of locations of simulated interaction between the geometry of the tool (20) and the anatomical volume (AV) at the location of the one point (P); and
for a second point of the tool path successive to the one point (P);
identifying a location of the second point relative to the anatomical data (65),
loading, from the tool data (69), the geometry of the tool (20) at the identified location of the second point,
at the identified location of the second point, identifying an intersection (I) between the geometry of the tool (20) and the anatomical volume (AV),
determining density values (DV) of the anatomical data (65) within the intersection at the location of the second point,
comparing coordinates of the determined density values (DV) to the interaction coordinates; and
disregarding any density values (DV) having coordinates that are identical to the interaction coordinates;
(B) the method further comprising, for the at least one point (P) of the tool path (TP):
disregarding any density values (DV) located beyond the intersection (I) between the geometry of the tool (20) and the anatomical volume (AV);
(C) wherein merging the path data (73) and the anatomical data (65) further includes merging the path data (73) and anatomical data (65) into a common coordinate system;
(D) wherein the cut plan data (79) comprises the tool path (TP) along which a robotic manipulator will move a tool (20) in an autonomous mode to interact with the anatomical volume (AV); and
(E) the method further comprising:
for each point (P) of the tool path (TP):
identifying the location of the point (P) relative to the anatomical data (65),
loading, from the tool data (69), the geometry of the tool (20) at the identified location,
at the identified location, identifying the intersection (I) between the geometry of the tool (20) and the anatomical volume (AV),
determining density values (DV) of the anatomical data (65) within the intersection (I),
setting the planned feed rate factor (FR) for the tool (20) based on the determined density values (DV), and
associating the planned feed rate factor (FR) with the point (P); and
wherein outputting the cut plan data (79) further comprises the tool path (TP) including the planned feed rate factor (FR) associated with each point (P) of the tool path (TP).

## Patentansprüche

1. Computerimplementiertes chirurgisches Planungsverfahren, umfassend:
Erhalten von anatomischen Daten (65), die eine Geometrie und Dichtewerte (DV) eines anatomischen Volumens (AV) umfassen;
Erhalten von Pfaddaten (73), die einen Werkzeugpfad (TP) umfassen, entlang dessen ein Robotermanipulator ein Werkzeug (20) bewegen wird, um mit dem anatomischen Volumen (AV) zu interagieren, wobei der Werkzeugpfad (TP) durch Punkte (P) definiert ist, die das Werkzeug (20) nacheinander durchlaufen wird;
Erhalten von Werkzeugdaten (69), die eine Geometrie des Werkzeugs (20) enthalten, das mit dem Werkzeugpfad (TP) interagieren wird;
Zusammenführen der Pfaddaten (73) und der anatomischen Daten (65); und
für mindestens einen Punkt (P) des Werkzeugpfads (TP):
Identifizieren einer Position des Punkts (P) relativ zu den anatomischen Daten (65),
Laden der Geometrie des Werkzeugs (20) an der identifizierten Position aus den Werkzeugdaten (69),
an der identifizierten Position, Identifizieren einer Schnittmenge (I) zwischen der Geometrie des Werkzeugs (20) und dem anatomischen Volumen (AV), und
Bestimmen von Dichtewerten (DV) der anatomischen Daten (65) innerhalb der Schnittmenge (I);
**dadurch gekennzeichnet, dass** das Verfahren für den mindestens einen Punkt (P) des Werkzeugpfads (TP) ferner umfasst:
Berechnen eines Werkzeugkontaktfaktors in Bezug auf eine Interaktion zwischen der Geometrie des Werkzeugs (20) und dem anatomischen Volumen (AV),
Einstellen eines geplanten Vorschubgeschwindigkeitsfaktors (FR) für das Werkzeug (20) basierend auf den bestimmten Dichtewerten (DV) und dem berechneten Werkzeugkontaktfaktor, und
Verknüpfen des geplanten Vorschubgeschwindigkeitsfaktors (FR) mit dem mindestens einen Punkt (P); und
Ausgeben von Schnittplandaten (79), die den Werkzeugpfad (TP) umfassen, der den geplanten Vorschubgeschwindigkeitsfaktor (FR) enthält, der mit dem mindestens einen Punkt (P) des Werkzeugpfads (TP) verknüpft ist.

2. Computerimplementiertes chirurgisches Planungsverfahren nach Anspruch 1,
wobei das Erhalten von anatomischen Daten (65) ferner ein Erhalten eines Knochenmodells umfasst, das mit dem anatomischen Volumen (AV) verknüpft ist; und optional,
wobei das Erhalten von Pfaddaten (73) ferner ein Erhalten des Werkzeugpfads (TP) umfasst, der basierend auf einem oder mehreren der Folgenden vorbestimmt ist:
einem geplanten Resektionsvolumen des Knochenmodells des anatomischen Volumens (AV); und
einer Geometrie eines Implantatmodells, das für das Knochenmodell ausgewählt wurde.

3. Computerimplementiertes chirurgisches Planungsverfahren nach Anspruch 1, wobei das Erhalten von anatomischen Daten (65) ferner ein Erhalten von Bildgebungsdaten umfasst, die Schichten des anatomischen Volumens (AV) enthalten.

4. Computerimplementiertes chirurgisches Planungsverfahren nach Anspruch 3, wobei das Erhalten von Bildgebungsdaten ferner ein Erhalten von DICOM-Daten umfasst, die Achsenabschnitt- und Steigungswerte, eine Schichtdicke und eine Patientenposition zu einem Bildgebungszeitpunkt umfassen.

5. Computerimplementiertes chirurgisches Planungsverfahren nach Anspruch 4, wobei an der identifizierten Position das Identifizieren der Schnittmenge (I) zwischen der Geometrie des Werkzeugs (20) und dem anatomischen Volumen (AV) ferner umfasst:
Identifizieren einer oder mehrerer Schichten der Bildgebungsdaten, die die Schnittmenge (I) zwischen der Geometrie des Werkzeugs (20) und dem anatomischen Volumen (AV) aufweisen.

6. Computerimplementiertes chirurgisches Planungsverfahren nach Anspruch 5, wobei das Bestimmen von Dichtewerten (DV) der anatomischen Daten (65) innerhalb der Schnittmenge (I) ferner umfasst:
für jede identifizierte Schicht, Bestimmen von Radiodichtewerten (DV) der Bildgebungsdaten, die sich innerhalb der Schnittmenge (I) befinden; und
Sammeln von Radiodichtewerten (DV), die sich innerhalb der Schnittmengen (I) jeder der einen oder mehreren identifizierten Schichten befinden.

7. Computerimplementiertes chirurgisches Planungsverfahren nach Anspruch 6, wobei das Einstellen des geplanten Vorschubgeschwindigkeitsfaktors (FR) für das Werkzeug (20) basierend auf den bestimmten Dichtewerten (DV) ferner umfasst:
Berechnen eines Knochenmineraldichte- (BMD-) Faktors des anatomischen Volumens (AV) relativ zu der identifizierten Position basierend auf den gesammelten Radiodichtewerten (DV); und
Einstellen des geplanten Vorschubgeschwindigkeitsfaktors (FR) für das Werkzeug (20) basierend auf dem berechneten BMD-Faktor.

8. Computerimplementiertes chirurgisches Planungsverfahren nach Anspruch 7, wobei:
das Erhalten von Bildgebungsdaten ferner ein Erhalten von CT-Schichten des anatomischen Volumens (AV) umfasst; und
die Geometrie des Werkzeugs (20) eine 3D-Geometrie des Werkzeugs (20) umfasst, und
für den mindestens einen Punkt des Werkzeugpfads (TP):
Laden der 3D-Geometrie des Werkzeugs (20) an der identifizierten Position, und
an der identifizierten Position, Identifizieren von CT-Schichten, für die es eine Querschnittsschnittmenge (I) zwischen der 3D-Geometrie des Werkzeugs (20) und dem anatomischen Volumen (AV) gibt;
für jede identifizierte CT-Schicht, Bestimmen einer Hounsfield-Einheit (HU) für jedes Pixel (PX) innerhalb der Querschnittsschnittmenge (I); und
Sammeln der Hounsfield-Einheiten (HU) von den Pixeln (PX) innerhalb der Querschnittsschnittmengen (I) jeder der identifizierten CT-Schichten.

9. Computerimplementiertes chirurgisches Planungsverfahren nach Anspruch 7, wobei:
das Sammeln der Radiodichtewerte (DV), die sich innerhalb der Schnittmenge (I) jeder der einen oder mehreren identifizierten Schichten befinden, ferner ein Identifizieren eines oder mehrerer eines mittleren, eines Median- und eines maximalen Radiodichtewerts (DV) aus den gesammelten Radiodichtewerten (DV) umfasst; und
das Berechnen des BMD-Faktors ferner ein Umwandeln des einen oder der mehreren des mittleren, des Median- und des maximalen Radiodichtewerts (DV) in den BMD-Faktor umfasst.

10. Computerimplementiertes chirurgisches Planungsverfahren nach Anspruch 9, ferner umfassend, für den mindestens einen Punkt (P) des Werkzeugpfads (TP), und nach dem Identifizieren von Schichten der Bildgebungsdaten, die die Schnittmenge (I) zwischen der Geometrie des Werkzeugs (20) und dem anatomischen Volumen (AV) aufweisen, für jede identifizierte Schicht, Berechnen des Werkzeugkontaktfaktors durch Berechnen eines Schnittverhältnisses in Bezug auf die Geometrie des Werkzeugs (20) innerhalb der Schnittmenge (I) relativ zu der Geometrie des Werkzeugs (20) außerhalb der Schnittmenge (I).

11. Computerimplementiertes chirurgisches Planungsverfahren nach Anspruch 10,
wobei das Umwandeln des einen oder der mehreren des mittleren, des Median- und des maximalen Radiodichtewerts (DV) in den BMD-Faktor ferner ein Multiplizieren des einen oder der mehreren des mittleren, des Median- und des maximalen Radiodichtewerts (DV) mit dem Schnittverhältnis (I) umfasst; oder
wobei, für jede identifizierte Schicht, eine Menge von Pixeln (PX) innerhalb der Geometrie des Werkzeugs (20) bestimmt wird, die Hounsfield-Einheiten (HU) aufweisen, die einen vorbestimmten Schwellenwert überschreiten.

12. Computerimplementiertes chirurgisches Planungsverfahren nach Anspruch 1, ferner umfassend:
Berechnen eines Knochenmineraldichte- (BMD-) Faktors des anatomischen Volumens (AV) relativ zu der identifizierten Position basierend auf den bestimmten Dichtewerten (DV); und
wobei das Einstellen des geplanten Vorschubgeschwindigkeitsfaktors (FR) für das Werkzeug (20) basierend auf den bestimmten Dichtewerten (DV) ferner ein Einstellen des geplanten Vorschubgeschwindigkeitsfaktors (FR) basierend auf dem berechneten BMD-Faktor umfasst.

13. Computerimplementiertes chirurgisches Planungsverfahren nach Anspruch 12, ferner umfassend:
Zugreifen auf eine Nachschlagetabelle, die Verknüpfungen zwischen vordefinierten BMD-Faktoren und vordefinierten Vorschubgeschwindigkeitsfaktoren (FR) definiert; und
wobei, für den mindestens einen Punkt (P) des Werkzeugpfads (TP), das Einstellen des geplanten Vorschubgeschwindigkeitsfaktors (FR) basierend auf dem berechneten BMD-Faktor ferner umfasst:
Identifizieren, in der Nachschlagetabelle, des vordefinierten BMD-Faktors, der dem berechneten BMD-Faktor am nächsten liegt; und
Einstellen des geplanten Vorschubgeschwindigkeitsfaktors (FR) basierend auf dem vordefinierten Vorschubgeschwindigkeitsfaktor (FR), der mit dem am nächsten liegenden identifizierten vordefinierten BMD-Faktor in der Nachschlagetabelle verknüpft ist.

14. Computerimplementiertes chirurgisches Planungsverfahren nach Anspruch 13, wobei, für den mindestens einen Punkt (P) des Werkzeugpfads (TP), das Einstellen des geplanten Vorschubgeschwindigkeitsfaktors (FR) basierend auf dem berechneten BMD-Faktor ferner umfasst:
Einstellen des geplanten Vorschubgeschwindigkeitsfaktors (FR) als einen maximalen Vorschubgeschwindigkeitsfaktor als Reaktion auf eine Bestimmung, dass der berechnete BMD-Faktor unter einem Mindestschwellenwert liegt; und/oder
Einstellen des geplanten Vorschubgeschwindigkeitsfaktors (FR) als einen minimalen Vorschubgeschwindigkeitsfaktor als Reaktion auf eine Bestimmung, dass der berechnete BMD-Faktor über einem Höchstschwellenwert liegt.

15. Computerimplementiertes chirurgisches Planungsverfahren nach Anspruch 1, wobei mindestens eine der folgenden Bedingungen (A bis E) erfüllt ist:
(A) das Verfahren umfasst ferner:
für einen Punkt (P) des Werkzeugpfads (TP), Speichern von Interaktionskoordinaten, die aus der Schnittmenge (I) zwischen der Geometrie des Werkzeugs (20) und dem anatomischen Volumen (AV) an der Position des einen Punkts (P) erhalten wurden, wobei die Interaktionskoordinaten indikativ für Positionen einer simulierten Interaktion zwischen der Geometrie des Werkzeugs (20) und dem anatomischen Volumen (AV) an der Position des einen Punkts (P) sind; und
für einen zweiten Punkt des Werkzeugpfads, der auf den einen Punkt (P) folgt:
Identifizieren einer Position des zweiten Punkts relativ zu den anatomischen Daten (65),
Laden der Geometrie des Werkzeugs (20) an der identifizierten Position des zweiten Punkts aus den Werkzeugdaten (69),
an der identifizierten Position des zweiten Punkts, Identifizieren einer Schnittmenge (I) zwischen der Geometrie des Werkzeugs (20) und dem anatomischen Volumen (AV),
Bestimmen von Dichtewerten (DV) der anatomischen Daten (65) innerhalb der Schnittmenge an der Position des zweiten Punkts,
Vergleichen von Koordinaten der bestimmten Dichtewerte (DV) mit den Interaktionskoordinaten; und
Ignorieren jeglicher Dichtewerte (DV), die Koordinaten aufweisen, die identisch mit den Interaktionskoordinaten sind;
(B) das Verfahren umfasst ferner, für den mindestens einen Punkt (P) des Werkzeugpfads (TP):
Ignorieren jeglicher Dichtewerte (DV), die jenseits der Schnittmenge (I) zwischen der Geometrie des Werkzeugs (20) und dem anatomischen Volumen (AV) liegen;
(C) wobei das Zusammenführen der Pfaddaten (73) und der anatomischen Daten (65) ferner ein Zusammenführen der Pfaddaten (73) und der anatomischen Daten (65) in ein gemeinsames Koordinatensystem umfasst;
(D) wobei die Schnittplandaten (79) den Werkzeugpfad (TP) umfassen, entlang dessen ein Robotermanipulator ein Werkzeug (20) in einem autonomen Modus bewegen wird, um mit dem anatomischen Volumen (AV) zu interagieren; und
(E) das Verfahren umfasst ferner:
für jeden Punkt (P) des Werkzeugpfads (TP):
Identifizieren der Position des Punkts (P) relativ zu den anatomischen Daten (65),
Laden der Geometrie des Werkzeugs (20) an der identifizierten Position aus den Werkzeugdaten (69),
an der identifizierten Position, Identifizieren der Schnittmenge (I) zwischen der Geometrie des Werkzeugs (20) und dem anatomischen Volumen (AV),
Bestimmen von Dichtewerten (DV) der anatomischen Daten (65) innerhalb der Schnittmenge (I),
Einstellen des geplanten Vorschubgeschwindigkeitsfaktors (FR) für das Werkzeug (20) basierend auf den bestimmten Dichtewerten (DV), und
Verknüpfen des geplanten Vorschubgeschwindigkeitsfaktors (FR) mit dem Punkt (P); und
wobei das Ausgeben der Schnittplandaten (79) ferner den Werkzeugpfad (TP) umfasst, der den geplanten Vorschubgeschwindigkeitsfaktor (FR) enthält, der mit jedem Punkt (P) des Werkzeugpfads (TP) verknüpft ist.

## Revendications

1. Procédé de planification chirurgicale mis en œuvre par ordinateur comprenant:
l'obtention de données anatomiques (65) comprenant une géométrie et des valeurs de densité (DV) d'un volume anatomique (AV);
l'obtention de données de trajet (73) comprenant un trajet d'outil (TP) le long de laquelle un manipulateur robotisé déplacera un outil (20) pour interagir avec le volume anatomique (AV), le trajet d'outil (TP) étant définie par des points (P) auxquels l'outil (20) passera successivement;
l'obtention de données d'outil (69) comprenant une géométrie de l'outil (20) qui interagira avec le trajet d'outil (TP);
la fusion des données de trajet (73) et les données anatomiques (65); et
pour au moins un point (P) du trajet d'outil (TP):
l'identification d'un emplacement du point (P) par rapport aux données anatomiques (65),
le chargement, à partir des données d'outil (69), de la géométrie de l'outil (20) à l'emplacement identifié,
à l'emplacement identifié, l'identification d'une intersection (I) entre la géométrie d'outil (20) et le volume anatomique (AV), et
la détermination des valeurs de densité (DV) des données anatomiques (65) dans l'intersection (I);
**caractérisé en ce que** le procédé comprend en outre, pour l'au moins un point (P) du trajet d'outil (TP):
le calcul d'un facteur de contact d'outil lié à une interaction entre la géométrie de l'outil (20) et le volume anatomique (AV),
la définition d'un facteur d'avance planifié (FR) pour l'outil (20) sur la base des valeurs de densité (DV) déterminées et du facteur de contact d'outil calculé, et
l'association du facteur d'avance planifié (FR) à l'au moins un point (P); et
la sortie de données du plan de coupe (79) comprenant le trajet d'outil (TP) y compris le facteur d'avance planifié (FR) associé à l'au moins un point (P) du trajet d'outil (TP).

2. Procédé de planification chirurgicale mis en œuvre par ordinateur selon la revendication 1,
dans lequel l'obtention des données anatomiques (65) comprend en outre l'obtention d'un modèle osseux associé au volume anatomique (AV); et, éventuellement,
dans lequel l'obtention des données de trajet (73) comprend en outre l'obtention du trajet d'outil (TP) prédéterminée sur la base d'un ou plusieurs des éléments suivants:
un volume de résection planifié du modèle osseux du volume anatomique (AV); et
la géométrie d'un modèle d'implant sélectionné pour le modèle osseux.

3. Procédé de planification chirurgicale mis en œuvre par ordinateur selon la revendication 1, dans lequel l'obtention des données anatomiques (65) comprend en outre l'obtention de données d'imagerie comprenant des coupes du volume anatomique (AV).

4. Procédé de planification chirurgicale mis en œuvre par ordinateur selon la revendication 3, dans lequel l'obtention des données d'imagerie comprend en outre l'obtention de données DICOM comprenant des valeurs d'interception et de pente, l'épaisseur de coupe et la position du patient au moment de l'imagerie.

5. Procédé de planification chirurgicale mis en œuvre par ordinateur selon la revendication 4, dans lequel, à l'emplacement identifié, l'identification de l'intersection (I) entre la géométrie de l'outil (20) et le volume anatomique (AV) comprend en outre:
l'identification d'une ou plusieurs coupes des données d'imagerie présentant l'intersection (I) entre la géométrie de l'outil (20) et le volume anatomique (AV).

6. Procédé de planification chirurgicale mise en œuvre par ordinateur selon la revendication 5, dans lequel la détermination des valeurs de densité (DV) des données anatomiques (65) dans l'intersection (I) comprend en outre:
pour chaque coupe identifiée, la détermination des valeurs de radiodensité (DV) des données d'imagerie situées dans l'intersection (I); et
la collecte des valeurs de radiodensité (DV) situées dans les intersections (I) de chacune des coupes identifiées.

7. Procédé de planification chirurgicale mise en œuvre par ordinateur selon la revendication 6, dans lequel la définition du facteur d'avance planifié (FR) pour l'outil (20) sur la base des valeurs de densité déterminées (DV) comprend en outre:
le calcul d'un facteur de densité minérale osseuse (DMO) du volume anatomique (VA) par rapport à l'emplacement identifié sur la base des valeurs de radiodensité (RD) collectées; et
la définition du facteur d'avance planifié (FR) pour l'outil (20) sur la base du facteur de DMO calculé.

8. Procédé de planification chirurgicale mis en œuvre par ordinateur selon la revendication 7, dans lequel:
l'obtention de données d'imagerie comprend en outre l'obtention de coupes CT du volume anatomique (AV); et
la géométrie de l'outil (20) comprend une géométrie 3D de l'outil (20), et
pour l'au moins un point du trajet de l'outil (TP):
le chargement de la géométrie 3D de l'outil (20) à l'emplacement identifié, et
à l'emplacement identifié, l'identification des coupes CT pour lesquelles il existe une intersection transversale (I) entre la géométrie 3D de l'outil (20) et le volume anatomique (AV);
ou pour chaque coupe CT identifiée, la détermination d'une unité Hounsfield (HU) pour chaque pixel (PX) dans l'intersection transversale (I); et
la collecte des unités Hounsfield (HU) à partir des pixels (PX) dans les intersections transversales (I) de chacune des coupes CT identifiées.

9. Procédé de planification chirurgicale mis en œuvre par ordinateur selon la revendication 7, dans lequel:
la collecte des valeurs de radiodensité (DV) situées dans l'intersection (I) de chacune de ladite ou desdites coupes identifiées comprend en outre l'identification d'une ou plusieurs valeurs parmi une valeur moyenne, une valeur médiane et une valeur maximale de radiodensité (DV) à partir des valeurs de radiodensité (DV) collectées; et
le calcul du facteur de DMO comprend en outre la conversion de la ou des valeurs parmi: la valeur moyenne, la valeur médiane et la valeur maximale de radiodensité (DV) en facteur DMO.

10. Procédé de planification chirurgicale mise en œuvre par ordinateur selon la revendication 9, comprenant en outre, pour l'au moins un point (P) de le trajet de l'outil (TP), et après avoir identifié les coupes des données d'imagerie présentant l'intersection (I) entre la géométrie de l'outil (20) et le volume anatomique (AV), pour chaque coupe identifiée, le calcul du facteur de contact de l'outil en calculant un rapport d'intersection lié à la géométrie de l'outil (20) à l'intérieur de l'intersection (I) par rapport à la géométrie de l'outil (20) à l'extérieur de l'intersection (I).

11. Procédé de planification chirurgicale mise en œuvre par ordinateur selon la revendication 10, dans lequel la conversion de la ou des valeurs parmi: la valeur moyenne, la valeur médiane et la valeur maximale de radiodensité (DV) en facteur de DMO comprend en outre la multiplication de la ou des valeurs parmi: la valeur moyenne, la valeur médiane et la valeur maximale de radiodensité (DV) par le rapport d'intersection (I); ou
dans lequel, pour chaque coupe identifiée, la détermination d'une quantité de pixels (PX) dans la géométrie de l'outil (20) ayant des unités Hounsfield (HU) dépassant un seuil prédéterminé.

12. Procédé de planification chirurgicale mis en œuvre par ordinateur selon la revendication 1, comprenant en outre:
le calcul d'un facteur de densité minérale osseuse (DMO) du volume anatomique (VA) par rapport à l'emplacement identifié sur la base des valeurs de densité déterminées (VD); et
dans lequel le définition du facteur d'avance planifié (FR) pour l'outil (20) sur la base des valeurs de densité déterminées (DV) comprend en outre la définition du facteur d'avance planifié (FR) sur la base du facteur de DMO calculé.

13. Procédé de planification chirurgicale mis en œuvre par ordinateur selon la revendication 12, comprenant en outre:
l'accès à une table de consultation définissant les associations entre les facteurs de DMO prédéfinis et les facteurs d'avance (FR) prédéfinis; et
dans lequel, pour l'au moins un point (P) du trajet d'outil (TP), la définition du facteur d'avance planifié (FR) sur la base du facteur DMO calculé comprend en outre:
l'identification, dans la table de consultation, du facteur de DMO prédéfini qui est le plus proche du facteur de planifié calculé; et
la définition du facteur d'avance planifié (FR) en fonction du facteur d'avance prédéfini (FR) associé au facteur de MDO prédéfini identifié le plus proche dans la table de consultation.

14. Procédé de planification chirurgicale mis en œuvre par ordinateur selon la revendication 13, dans lequel, pour l'au moins un point (P) du trajet d'outil (P), la définition du facteur d'avance planifié (FR) sur la base du facteur de DMO calculé comprend en outre:
la définition du facteur d'avance planifié (FR) comme étant un facteur d'avance maximal en réponse à la détermination que le facteur de DMO calculé est inférieur à un seuil minimal; et/ou
la définition du facteur d'avance planifié (FR) comme étant un facteur d'avance minimal en réponse à la détermination que le facteur de MDO calculé est supérieur à un seuil maximal.

15. Procédé de planification chirurgicale mis en œuvre par ordinateur selon la revendication 1,
dans lequel au moins l'une des conditions suivantes (A à E) est remplie:
(A) le procédé comprenant en outre:
pour un point (P) du trajet d'outil (TP), le stockage des coordonnées d'interaction obtenues à partir de l'intersection (I) entre la géométrie de l'outil (20) et le volume anatomique (AV) à l'emplacement dudit point (P), les coordonnées d'interaction indiquant les emplacements d'interaction simulée entre la géométrie de l'outil (20) et le volume anatomique (AV) à l'emplacement dudit point (P); et
pour un deuxième point du trajet d'outil successif audit point (P);
l'identification d'un emplacement du deuxième point par rapport aux données anatomiques (65),
le chargement, à partir des données d'outil (69), de la géométrie de l'outil (20) à l'emplacement identifié du deuxième point,
à l'emplacement identifié du deuxième point, l'identification d'une intersection (I) entre la géométrie de l'outil (20) et le volume anatomique (AV),
la détermination des valeurs de densité (DV) des données anatomiques (65) dans l'intersection (I) à l'emplacement du deuxième point,
la comparaison des coordonnées des valeurs de densité déterminées (DV) avec les coordonnées d'interaction; et
le rejet de toutes les valeurs de densité (DV) dont les coordonnées sont identiques aux coordonnées d'interaction;
(B) le procédé comprenant en outre, pour l'au moins un point (P) du trajet d'outil (TP):
le rejet de toutes les valeurs de densité (DV) situés au-delà de l'intersection (I) entre la géométrie de l'outil (20) et le volume anatomique (AV);
(C) dans lequel la fusion des données de trajet (73) et des données anatomiques (65) comprend en outre la fusion des données de trajet (73) et des données anatomiques (65) dans un système de coordonnées commun;
(D) dans lequel les données du plan de coupe (79) comprennent le trajet d'outil (TP) le long duquel un manipulateur robotisé déplacera un outil (20) en mode autonome pour interagir avec le volume anatomique (AV); et
(E) le procédé comprenant en outre:
pour chaque point (P) du trajet d'outil (TP):
l'identification d'un emplacement du point (P) par rapport aux données anatomiques (65),
le chargement, à partir des données d'outil (69), de la géométrie de l'outil (20) à l'emplacement identifié,
à l'emplacement identifié, l'identification de l'intersection (I) entre la géométrie de l'outil (20) et le volume anatomique (AV),
la détermination des valeurs de densité (DV) des données anatomiques (65) dans l'intersection (I),
la définition du facteur d'avance planifié (FR) pour l'outil (20) sur la base des valeurs de densité déterminée (DV), et
l'association du facteur d'avance planifié (FR) au point (P); et
dans lequel la sortie des données de plan de coupe (79) comprenant en outre le trajet d'outil (TP) y compris le facteur d'avance planifié (FR) associé à chaque point (P) du trajet d'outil (TP).
